(19) Europäisches Patentamt
European Patent Office
Office européen des brevets

(11) Publication number: **0 137 963**
**B1**

(12) # EUROPEAN PATENT SPECIFICATION

(45) Date of publication of patent specification: 28.09.88

(21) Application number: 84109807.2

(22) Date of filing: 17.08.84

(51) Int. Cl.$^4$: **C 07 C 49/813**, C 07 C 49/84, C 07 C 62/38, C 07 C 79/36, C 07 C 147/06, C 07 C 149/273, C 07 D 317/62, A 01 N 35/06

(54) **Certain 2-(2-substituted Benzoyl)-1,3-Cyclohexanediones.**

(30) Priority: 31.07.84 US 634408
07.03.84 US 587331
16.09.83 US 532869

(43) Date of publication of application:
24.04.85 Bulletin 85/17

(45) Publication of the grant of the patent:
28.09.88 Bulletin 88/39

(84) Designated Contracting States:
AT BE CH DE FR GB IT LI NL SE

(56) References cited:
EP-A-0 017 195
EP-A-0 090 262
US-A-4 227 919
US-A-4 256 658
US-A-4 283 348

**The file contains technical information submitted after the application was filed and not included in this specification**

(73) Proprietor: STAUFFER CHEMICAL COMPANY
Westport Connecticut 06881 (US)

(72) Inventor: Michaely, William James
3161 Birmingham Drive
Richmond, CA.94806 (US)
Inventor: Kraatz, Gary Wayne
1423 Bing Drive
San Jose, CA.95129 (US)

(74) Representative: Kraus, Walter, Dr. et al
Patentanwälte Kraus, Weisert & Partner
Thomas-Wimmer-Ring 15
D-8000 München 22 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 137 963 B1

## Description

This invention relates to 2-(2-substituted benzoyl)-cyclohexane-1,3-diones, a method of controlling undesirable vegetation, and herbicidal composition.

Compounds having the structural formula

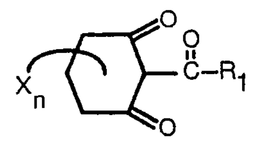

wherein x can be an alkyl, n can be 0, 1 or 2, and $R_1$ can be phenyl or substituted phenyl are described in Japanese patent application 84632—1974 as being intermediates for the preparation of herbocidial compounds of the formula

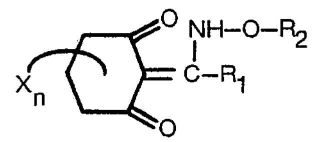

wherein $R_1$, X, and n are as defined above and $R_2$ is alkyl, alkenyl, or alkynyl. Sprcifically taught herbicidal compounds of this latter group are those where n is 2, X is 5,5-dimethyl, $R_2$ is allyl and $R_1$ is phenyl, 4-chlorophenyl or 4-methoxyphenyl.

The precursor intermediates for these three specifically taught compounds have no or almost no herbicidal activity.

In EP—A2—0 090 262 (with priorities from 25.03.82 and 09.02.83, published 05.10.83) herbicidally active 2-(2-substituted benzoyl)-cyclohexane-1,3-diones are described. The compounds described here are new with respect to D1 and were all described in the first basic U.S. application, Ser. No. 532 869 of September 16, 1983, the priority of which is claimed for this case.

In contrast to the compounds known from Japanese patent application 84632—1974, the compounds of this invention have exceptional herbicidal activity. Applicant's compounds must have chlorine, bromine, iodine or alkoxy substitution in the 2-position of the phenyl moiety of their compounds to obtain the exceptional herbicidal activity. Chlorine is the preferred substituent. The exact reason why such a substitution imparts exceptional herbicidal activity to the compound is not fully understood.

The compounds of this invention have the structural formula

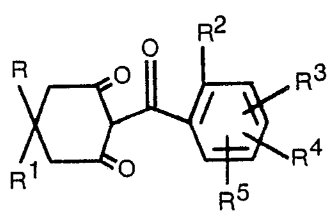

wherein

R and $R^1$ are hydrogen, $C_1$—$C_4$ alkyl, $R^aOC(O)$—, where $R^a$ is $C_1$—$C_4$ alkyl, $R_2$ is chlorine, bromine, iodine or $C_1$—$C_4$ alkoxy, and $R^3$ and $R^4$ and $R^5$ independently are (1) hydrogen, (2) halogen, (3) $C_1$—$C_4$ alkyl, (4) $C_1$—$C_4$ alkoxy, (5) nitro, (6) $C_1$—$C_4$ haloalkyl, (7) $R^bSO_n$— wherein $R^b$ is $C_1$—$C_4$ alkyl and n is the integer 0, 1 or 2, (8)

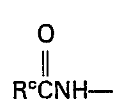

wherein $R^c$ is $C_1$—$C_4$ alkyl, or (9) $R^3$ and $R^4$ together can form a ring structure with two adjacent carbon atoms of the phenyl ring,

and their salts, with the proviso that compounds having the structural formula

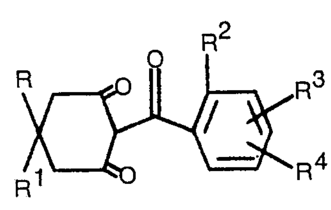

wherein
R and $R^1$ are hydrogen or $C_1$—$C_4$ alkyl,
$R^2$ is chlorine, bromine or iodine,
$R^3$ is hydrogen or halogen, and
$R^4$ is hydrogen, $C_1$—$C_4$ alkyl, $C_1$—$C_4$ alkoxy, nitro, or trifluoromethyl
are excluded.

Preferably R and $R^1$ are methyl or isopropyl, most preferably R and $R^1$ are hydrogen. Preferably $R^2$ is methoxy, most preferably $R^2$ is chlorine, bromine or methoxy. Preferably $R^3$, $R^4$ and $R^5$ are chlorine or bromine, methyl, methoxy or trifluoromethyl. If $R^3$, $R^4$ and $R^5$ are $R^bSO_n$—, $R^b$ is preferably methyl, and n is preferably 2.

Most preferably, $R^3$ is chlorine, hydrogen, dimethylamino or methoxy. Preferably $R^4$ is hydrogen, chlorine, nitro, $SO_2CH_3$, $SO_2N(CH_3)_2$ or $CF_3$. Preferably $R^5$ is hydrogen.

The most preferred compound is the compound having the above-mentioned formula wherein R is hydrogen, $R^1$ is hydrogen, $R^2$ is chlorine, $R^3$ is hydrogen, $R^4$ is 4-$CH_3SO_2$ and $R^5$ is hydrogen and its salts.

This invention also relates to a method controlling undesirable vegetation comprising applying to the area where control is desired, a herbically effective amount of a compound mentioned above.

The compounds of this invention can have the following four structural formulae because of tautomerism:

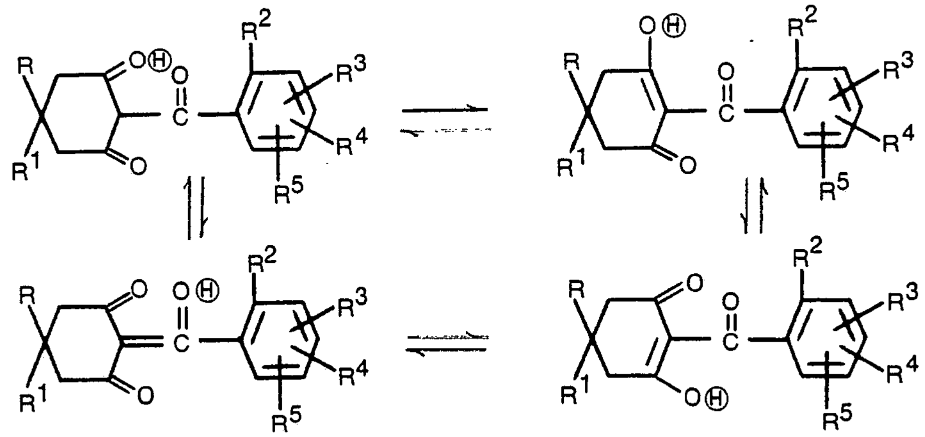

wherein R, $R^1$, $R^2$, $R^3$, $R^4$ and $R^5$ are as defined above.

The circled proton on each oif the four tautomers is reasonably labile. These protons are acidic and can be removed by any base to give a salt having an anion of the following four resonance forms:

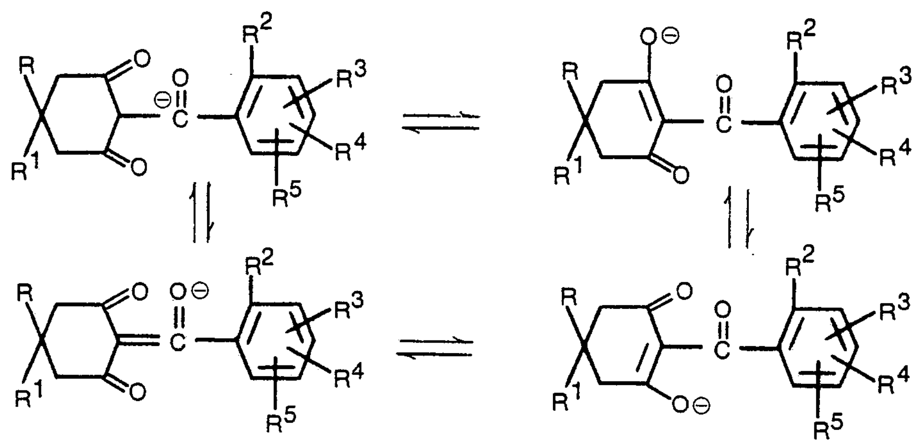

wherein R, $R^1$, $R^2$, $R^3$, $R^4$ and $R^5$ are as defined above.

Examples of cations of these bases are inorganic cations such as alkali metals, e.g. lithium, sodium, and potassium alkaline earth metals, e.g. barium, magnesium, calcium and strontium or organic cations such as substituted ammonium, sulfonium or phosphonium wherein the substituted is an aliphatic or aromatic group.

The term "aliphatic group" is used herein in a broad sense to cover a large class of organic groups characterized by being derived from (1) an acylic (open-chain structure) of the paraffin, olefin and acetylene hydrocarbon series and their derivatives or (2) alicyclic compounds. The aliphatic group can have from 1 to 10 carbon atoms.

The term "aromatic group" is used herein in a broad sense to distinguish from the aliphatic group and includes a group derived from (1) compounds having 6 to 20 carbon atoms and characterized by the presence of at least one benzene ring, including monocyclic, bicyclic and polycyclic hydrocarbons and their derivatives and (2) heterocyclic compounds having 5 to 19 carbon atoms which are similar in structure and are characterized by having an unsaturated ring structure containing at least one atom other than carbon such as nitrogen, sulfur and oxygen and derivatives of these heterocyclic compounds.

In the above description of the compounds of this invention alkyl and alkoxy include both straight and branched configurations; for example, methy, ethyl, n-propyl, isopropyl, n-butyl, sec-butyl, isobutyl, and tert-butyl.

The compounds of this invention and their salts are active herbicides of a general type. That is, they are herbicidally effective against a wide range of plant species. The method of controlling undesirable vegetation of the present invention comprises applying a herbicidally effective amount of the above-described compounds to the area where control is desired.

The compounds of the present invention can be prepared by the following general method.

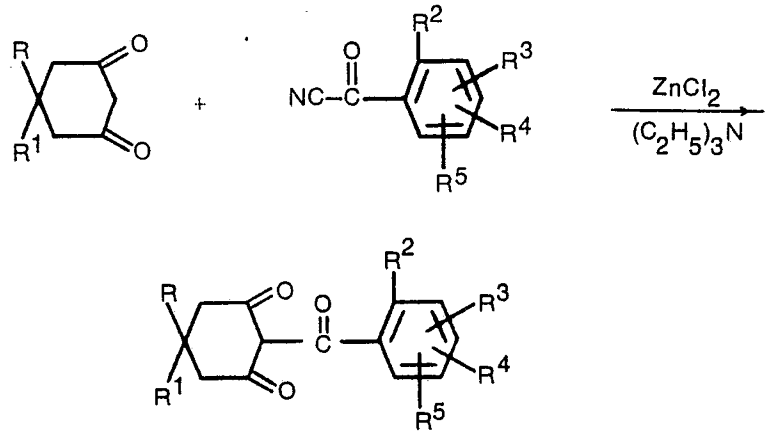


Generally, mole amounts of the dione and substituted benzoyl cyanide are used, along with a slight mole excess of zinc chloride. The two reactants and the zinc chloride are combined in a solvent such as methylene chloride. A slight mole excess of triethylamine is slowly added to the reaction mixture with cooling. The mixture is stirred at room temperature for 5 hours.

The reaction product is worked up by conventional techniques.

The above-described substituted benzoyl cyanide can be prepared according to the teaching of T. S. Oakwood and C. A. Weisgerber, *Organic Synthesis Collected,* Vol. III, pp. 122 (1955).

The following example teaches the synthesis of a representative compound of this invention.

Example 1

2-(2,4-Dichlorobenzoyl)-cyclohexane-1,3-dione

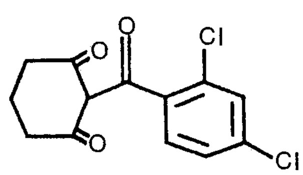

1,3-Cyclohexanedione [11.2 grams (g), 0.1 mole] 20.0 g (0.1 mole) 2,4-dichlorobenzoyl cyanide and 13.6 g (0.11 mole) anhydrous, powdered zinc chloride were combined in 100 milliliters (ml) methylene chloride. Triethylamine (10.1 g, 0.12 mole) was slowly added with cooling. The reaction mixture was stirred at room temperature for 5 hours and then poured into 2N hydrochloric acid. The aqueous phase was discarded and the organic phase was washed with 150 ml 5% $Na_2CO_3$ four times. The aqueous washings were combined and acidified with HCl, extracted with methylene chloride, dried and concentrated to yield 25.3 g of crude product. The crude product was dissolved in ether and stirred with 250 ml of 5% copper (II) acetate. The resulting copper salt was filtered, washed with ether and stirred with 6N hydrochloric acid to destroy the salt. The extract was washed with ether to yield 22.15 grams of the desired product m.p. 138—140°C (77.7% yield). The structure was confirmed by instrumental analysis.

The following is a table of certain selected compounds that are preparable according to the procedure described hereto. Compound numbers are assigned to each compound and are used throughout the remainder of the application.

TABLE I

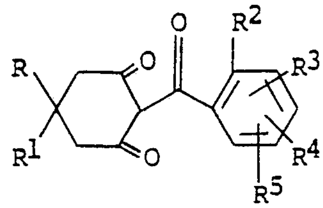

| Compound Number | R | $R^1$ | $R^2$ | $R^3$ | $R^4$ | $R^5$ | $N_D^{30}$ or m.p. °C |
|---|---|---|---|---|---|---|---|
| 1 | $CH_3$ | $CH_3$ | $CH_3O$ | H | H | H | 104-108 |
| 2 | H | H | Cl | H | (a) | H | |
| 3 | H | H | Br | H | H | (b) | |
| 4 | H | H | $CH_3O$ | 3-$CH_3O$ | H | H | 75-79 |
| 5 | H | H | $CH_3O$ | H | H | 5-$CH_3O$ | 89-92 |
| 6 | H | H | Br | H | 4-$CH_3O$ | 5-$CH_3O$ | 92-96 |
| 7 | H | H | Br | H | (4)—$OCH_2O$—(5) | | 63-68 |
| 8 | $CH_3$ | $CH_3$ | Cl | 3-Cl | 4-Cl | 5-Cl | 137-139 |
| 9 | H | H | Cl | 3-Cl | 4-Cl | 5-Cl | 106-110 |
| 10 | $CH(CH_3)_2O-\overset{O}{\overset{\|}{C}}-$ | H | Cl | H | 4-Cl | H | |
| 11 | $CH_3O-\overset{O}{\overset{\|}{C}}-$ | H | Cl | H | 4-Cl | H | |
| 12 | sodium salt of Compound No. 23 | | | | | | 85-94 |
| 13 | isopropylamine salt of Compound No. 23 | | | | | | 160-165 |
| 14 | triethylamine salt of Compound No. 23 | | | | | | 82-85 |
| 15 | potassium salt of Compound No. 23 | | | | | | 107-106 |
| 16 | triethanolammonium salt of Compound No. 39 | | | | | | |
| 17 | sodium salt of Compound No. 43 | | | | | | 60-63 |

TABLE I

(continued)

| Compound Number | R | $R^1$ | $R^2$ | $R^3$ | $R^4$ | $R^5$ | $N_D^{30}$ or m.p. °C |
|---|---|---|---|---|---|---|---|
| 18 | H | H | Cl | H | 4-$CH_3SO_2$- | H | |
| 19 | $CH_3$ | $CH_3$ | Cl | H | 4-$CH_3SO_2$- | H | |
| 20 | H | H | Cl | 3-$CH_3$ | 4-Cl | H | |
| 21 | H | H | Cl | H | 4-$C_2H_5SO_2$- | H | 98-100 |
| 22 | H | H | Cl | 3-Cl | 4-$C_2H_5SO_2$- | H | 98-109 |
| 23 | H | H | Cl | H | 4-$C_3H_7SO_2$- | H | semi-solid |
| 24 | H | H | Cl | 3-$OCH_3$ | 4-$CH_3SO_2$- | H | 48-59 |
| 25 | $CH_3$ | $CH_3$ | Cl | H | 4-n-$C_3H_7SO_2$- | H | semi-solid |
| 26 | H | H | Cl | 3-Cl | 4-n-$C_3H_7SO_2$- | H | 145-148.5 |
| 27 | H | H | Cl | 3-$C_2H_5S$ | 4-$C_2H_5SO_2$- | H | oil |
| 28 | $CH_3$ | $CH_3$ | Cl | H | 4-$C_2H_5SO_2$- | H | 103-108 |
| 29 | $CH_3$ | $CH_3$ | Cl | 3-Cl | 4-$C_2H_5SO_2$- | H | 108-111 |
| 30 | i-$C_3H_7$ | H | Cl | H | 4-$C_2H_5SO_2$- | H | brown gum |
| 31 | H | H | Cl | 3-Cl | 4-$CH_3SO_2$- | H | 145-154 |
| 32 | i-$C_3H_7$ | H | Cl | 3-Cl | 4-$CH_3SO_2$- | H | |
| 33 | H | H | Br | H | 4-$CH_3SO_2$ | H | brown gum |
| 34 | H | H | Cl | H | 4-i-$C_3H_7SO_2$- | H | |
| 35 | H | H | Cl | H | 4-$CH_3S$- | H | 70-77 |
| 36 | H | H | Cl | 3-Cl | 4-$CH_3S$- | H | |
| 37 | $CH_3$ | $CH_3$ | Cl | 3-Cl | 4-$C_2H_5SO_2$- | H | 120-123 |
| 38 | H | H | Cl | H | 4-n-$C_4H_9SO_2$- | H | |
| 39 | $CH_3$ | $CH_3$ | Cl | H | 4-n-$C_4H_9SO_2$- | H | |
| 40 | H | H | Cl | 3-$OC_2H_5$- | 4-$CH_3SO_2$- | H | semi-solid |
| 41 | H | H | Cl | 3-$OCH_3$ | 4-n-$C_3H_7SO_2$- | H | golden gum |
| 42 | $CH_3$ | $CH_3$ | Cl | H | 4-i-$C_3H_7SO_2$- | H | 125-128 |

* = Prepared in example.

(a) = 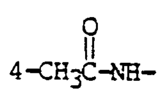 and (b) = 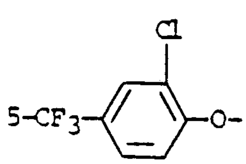

Herbicidal Screening Tests

As previously mentioned, the herein described compounds produced in the above-described manner are phytotoxic compounds which are useful and valuable in cotrolling various plant species. Selected compounds of this invention were tested as herbicides in the following manner.

*Pre-emergence herbicide test.* On the day preceding treatment, seeds of eight different weed species are planted in loamy sand soil in individual rows using one species per row across the width of a flat. The seeds used are green foxtail (FT) *(Setaria viridis),* watergrass (WG) *Echinochloa crusgalli),* wild oat (WO) *(Avena fatua),* annual morningglory (AMG) *(Ipomoea lacunosa),* velvetleaf (VL) *Abutilon theophrasti),*

Indian mustard (MD) *(Brassica juncea)*, redroot pigweed (PW) *Amaranthus retroflexus)* or curly dock (CD) *(Rumex crispus)*, and yellow nutsedge (YNG) *(Cyperus esculentus)*. Ample seeds are planted to give about 20 to 40 seedlings per row, after emergence, depending upon the size of the plants.

Using an analytical balance, 600 milligrams (mk) of the compound to be tested are weighed out on a piece of glassine weighing paper. The paper and compound are placed in a 60 milliter (ml) wide-mouth clear bottle and dissolved in 45 ml of acetone or substituted solvent. Eighteen ml of this solution are transferred to a 60-ml wide-mouth clear bottle and diluted with 22 ml of a water and acetone mixture (19:1) containing enough polyoxyethylene sorbitan monolaurate emulsifier to give a final solution of 0.5% (v/v). The solution is then sprayed on a seeded flat on a linear spray table calibrated to deliver 80 gallons per acre (748 L/ha). The application rate is 4 lb/acre (4.48 Kg/ha).

After treatment, the flats are placed in the greenhouse at a temperature of 70 to 80°F and watered by sprinkling. Two weeks after treatment, the degree of injury or control is determined by comparison with untreated check plants of the same age. The injury rating from 0 to 100% is recorded for each species as percent control with 0% representing no injury and 100% reprsenting complete control.

The results of the tests are shown in the following Table II.

TABLE II

Pre-Emergence Herbicidal Activity
Application Rate — 4.48 kg/ha

| Cmpd. No. | FT | WG | WO | AMG | VL | MD | PW | CD | YNG |
|---|---|---|---|---|---|---|---|---|---|
| 1 | 40 | 15 | 0 | 0 | 0 | 10 | | 0 | 30 |
| 2 | 40 | 80 | 10 | 20 | 60 | 50 | | 30 | 75 |
| 3 | 65 | 75 | 0 | 65 | 100 | 95 | | 75 | 55 |
| 4 | 100 | 95 | 55 | 10 | 80 | 75 | | 85 | 95 |
| 5 | 20 | 20 | 20 | 10 | 40 | 40 | | 50 | 20 |
| 6 | 75 | 95 | 20 | 20 | 100 | 95 | | 75 | 10 |
| 7 | 90 | 85 | 20 | 0 | 100 | 85 | | 75 | 30 |
| 8 | 0 | 0 | 0 | 20 | 100 | 100 | | 100 | 0 |
| 9 | 40 | 0 | 0 | 20 | 100 | 60 | | 90 | - |
| 10 | 80 | 10 | 0 | 0 | 0 | 0 | | 0 | 90 |
| 11 | 60 | 0 | 0 | 0 | 0 | 0 | | 0 | 90 |
| 12 | 90 | 100 | 0 | 60 | 100 | 100 | | 100 | 95 |
| 13 | 100 | 100 | 0 | 60 | 100 | 100 | | 100 | 100 |
| 14 | 20 | 60 | 0 | 100 | 100 | 100 | | 100 | 20 |
| 15 | 60 | 70 | 20 | 100 | 100 | 100 | | 100 | 60 |
| 16 | 60 | 0 | 0 | 0 | 0 | 0 | | 0 | 40 |
| 17 | 100 | 100 | 50 | 10 | 60 | 20 | | 90 | 100 |
| 18 | 100 | 100 | 90 | 100 | 100 | 100 | | 85 | 100 |
| 19 | 90 | 100 | 60 | 100 | 100 | 100 | | 90 | 100 |
| 20 | 100 | 100 | 90 | 100 | 100 | 100 | | 85 | 100 |
| 21 | 90 | 95 | 0 | 20 | 20 | 40 | | 40 | 100 |
| 22 | 95 | 100 | 85 | 75 | 100 | 100 | | 90 | 98 |
| 23 | 95 | 100 | 50 | 50 | 100 | 100 | | 90 | 98 |
| 24 | 100 | 100 | 75 | 100 | 100 | 100 | | 95 | 95 |
| 25 | 100 | 100 | 95 | 75 | 100 | 100 | | 100 | 80 |
| 26 | 100 | 100 | 40 | 65 | 100 | 100 | | 100 | 95 |
| 27 | 100 | 100 | 98 | 100 | 100 | 100 | | 100 | 90 |

TABLE II

(continued)

| Cmpd. No. | FT | WG | WO | AMG | VL | MD | PW | CD | YNG |
|---|---|---|---|---|---|---|---|---|---|
| 28 | 100 | 100 | 80 | 100 | 100 | 100 | | 100 | 90 |
| 29 | 100 | 100 | 75 | 100 | 100 | 100 | | 100 | 98 |
| 36 | 100 | 100 | 60 | 80 | 100 | 100 | | 75 | 90 |
| 37 | 100 | 100 | 70 | 80 | 100 | 100 | | 80 | 100 |
| 38 | 70 | 100 | 0 | 50 | 100 | 100 | | 100 | 80 |
| 39 | 40 | 90 | 25 | 10 | 100 | 100 | | 100 | 10 |
| 40 | 100 | 100 | 90 | 100 | 100 | 100 | | 85 | - |
| 41 | 100 | 100 | 80 | 100 | 100 | 100 | | 90 | - |

- = Species did not germinate for some reason.
A blank indicates that the weed was not tested.

*Post-Emergence Herbicide Test:* This test is conducted in an identical manner to the testing procedure for the pre-emergence herbicide test, except the seeds of the eight different weed species are planted 0-12 days before treatment. Also, watering of the treated flats is confined to the soil surface and not to the foliage of the sprouted plants.

The results of the post-emergence herbicide test are reported in Table III.

TABLE III

Post-Emergence Herbicidal Activity
Application Rate — 4.48 kg/ha

| Cmpd. No. | FT | WG | WO | AMG | VL | MD | PW | CD | YNG |
|---|---|---|---|---|---|---|---|---|---|
| 1 | 45 | 30 | 0 | 40 | 70 | 65 | | 0 | 45 |
| 2 | 75 | 80 | 30 | 65 | 50 | 45 | | 85 | 85 |
| 3 | 75 | 60 | 60 | 75 | 100 | 70 | | 80 | 25 |
| 4 | 85 | 85 | 85 | 75 | 85 | 65 | | 65 | 85 |
| 5 | 75 | 50 | 20 | 10 | 0 | 0 | | 20 | 40 |
| 6 | 60 | 60 | 20 | 40 | 40 | 70 | | 100 | 40 |
| 7 | 60 | 25 | 20 | 40 | 90 | 65 | | 20 | 40 |
| 8 | 0 | .0 | 0 | 20 | 20 | 20 | | 0 | 0 |
| 9 | 0 | 0 | 0 | 30 | 40 | 20 | | 80 | 0 |
| 10 | 40 | 40 | 20 | 0 | 0 | 0 | | 5 | 80 |
| 11 | 90 | 70 | 40 | 20 | 60 | 80 | | 20 | 65 |
| 12 | 80 | 85 | 0 | 60 | 90 | 90 | | 100 | 80 |
| 13 | 90 | 90 | 10 | 100 | 100 | 100 | | 100 | 90 |
| 14 | 80 | 100 | 0 | 60 | 100 | 100 | | 100 | 100 |
| 15 | 100 | 100 | 0 | 60 | 100 | 100 | | 100 | 100 |
| 16 | 40 | 40 | 20 | 10 | 10 | 10 | | 90 | 40 |
| 17 | 40 | 40 | 40 | 80 | 100 | 100 | | 60 | 60 |

TABLE III

(continued)

| Cmpd. No. | FT | WG | WO | AMG | VL | MD | PW | CD | YNG |
|---|---|---|---|---|---|---|---|---|---|
| 18 | 80 | 80 | 95 | 100 | 100 | 100 | | 100 | 90 |
| 19 | 40 | 40 | 40 | 60 | 60 | 60 | | 80 | 90 |
| 20 | 80 | 80 | 80 | 95 | 100 | 100 | | 100 | 90 |
| 21 | 60 | 70 | 10 | 70 | 10 | 10 | | 20 | 80 |
| 22 | 100 | 100 | 90 | 100 | - | 100 | | 100 | 50 |
| 23 | 100 | 100 | 100 | 100 | - | 100 | | 100 | 70 |
| 24 | 90 | 85 | 90 | 85 | - | 100 | | 100 | 80 |
| 25 | 100 | 85 | 100 | 75 | 100 | 100 | | 100 | 35 |
| 26 | 70 | 80 | 20 | 100 | 100 | 100 | | 35 | 40 |
| 27 | 75 | 75 | 90 | 70 | 90 | 70 | | 40 | 40 |
| 28 | 100 | 70 | 100 | 55 | 90 | 95 | | 100 | 40 |
| 29 | 100 | 75 | 100 | 90 | 95 | 95 | | 100 | 20 |
| 36 | 100 | 100 | 95 | 100 | 100 | 100 | | 55 | 30 |
| 37 | 100 | 95 | 75 | 100 | 100 | 100 | | 85 | 40 |
| 38 | 100 | 100 | 30 | 95 | 80 | 100 | | 80 | 40 |
| 39 | 80 | 80 | 75 | 75 | 100 | 95 | | 95 | 25 |
| 40 | 80 | 80 | 80 | 90 | 90 | 80 | | 90 | 80 |
| 41 | 80 | 85 | 75 | 85 | 80 | 95 | | 100 | 50 |

*Pre-Emergence Multi-Weed Herbicide Test*

Several compounds were evaluated at an application of 2 lb/acre (2.24 kg/ha) for pre-emergence activity against a larger number of weed species.

The process was generally similar to the pre-emergence herbicide test described above except except that only 300 milligrams of test compound were weighed out and the application rate was 40 gallons per acre.

Redroot pigweed (PW) and curly dock (CD) were elimiated in this test and the following weed species were added.

| | | |
|---|---|---|
| *Grasses:* | | |
| downybrame | *Bramus tectorum* | (DB) |
| annual ryegrass | *Lolium multiflorum* | (ARG) |
| rox-orange sorghum | *Sorghum bicolor* | (SHC) |
| hemp sesbania | *Sesbania exaltata* | (SESB) |
| nightsaade | *Solanum sp.* | (SP) |
| cocklebur | *Xattiium sp.* | (CB) |

The results of the test are shown in Table IV.

TABLE IV

Pre-Emergence Multi-weed Herbicide Test

| Cmpd. No. | DB | FT | ARG | WG | SHC | WO | BSG | AMG | SESB | VL | SP | MD | YNS | CB |
|---|---|---|---|---|---|---|---|---|---|---|---|---|---|---|
| 30 | 20 | 90 | 25 | 98 | 80 | 60 | 90 | 45 | 90 | 45 | 20 | 85 | 85 | 15 |
| 31 | 65 | 98 | 98 | 100 | 100 | 60 | 95 | 100 | 100 | 100 | 80 | 100 | 95 | 100 |
| 32 | 65 | 100 | 70 | 100 | 100 | 65 | 95 | 100 | 95 | 70 | 65 | 100 | 90 | - |
| 33 | 98 | 100 | 100 | 100 | 100 | 90 | 98 | 90 | 100 | 90 | 60 | 100 | 95 | 80 |
| 34 | 60 | 20 | 100 | 100 | 100 | 80 | 95 | 35 | 40 | 100 | 20 | 90 | 100 | 40 |
| 35 | 100 | 100 | 100 | 100 | 100 | 100 | 100 | 100 | 90 | 100 | 60 | 100 | 100 | 20 |

The compounds of the present invention are useful as herbicides, especially as pre-emergence herbicides, and can be applied in a variety of ways at various concentrations. In practise, the compounds herein defined are formulated into herbicidal compositions, by admixture, in herbicidally effective amounts, with the adjuvants and carriers normally employed for facilitating the dispersion of active ingredients for agricultural applications, recognizing the fact that the formulation and mode of application of a toxicant may affect the activity of the materials in a given application. Thus, these active herbicidal compounds may be formulated as granules of relatively large particle size, as wettable powders, as emulsifiable concentrates, as powdery dusts, as solutions or as any of several other known types of formulations, depending upon the desired mode of application. Preferred formulations for pre-emergence herbicidal applications are wettable powders, emulsifiable concentrates and granules. These formulations may contain as little as about 0.5% to as much as about 95% or more by weight of active ingredient. A herbicidally effective amount depends upon the nature of the seeds or plants to be controlled and the rate of application varies from about 0.05 to approximately 25 pounds per acre, prefeably from about 0.1 to about 10 pounds per acre.

Wettable powders are in the form of finely divided particles which disperse readily in water or other dispersants. The wettable powder is ultimately applied to the soil either as a dry dust or as a dispersion in water or other liquid. Typical carriers for wettable powders include fuller's earth, kaolin clay, silicas and other readily wet organic or inorganic diluents. Wettable powders normally arew prepared to contain about 5% to about 95% of the active ingredient and usually also contain a small amount of wetting, dispersing, or emulsifying agent to facilitate wetting and dispersion.

Emulsifiable concentrates are homogeneous liquid compositions which are dispersible in water or other dispersant, and may consist entirely of the active compound with a liquid or solid emulsifying agent, or may also contain a liquid carrier, such as xylene, heavy aromatic naphthal, isophorone and other nonvolatile organic solvents. For herbicidal application, these concentrates are dispersed in water or other liquid carrier and normally applied as a spray to the area to be treated. The percentage by weight of the essential active ingredient may vary according to the manner in which the composition is to be applied, but in general comprises about 0.5% to 95% of active ingredient by weight of the herbicidal composition.

Granular formulations wherein the toxicant is carried on relatively coarse particles, are usually applied without dilution to the area in which suppression of vegetation is desired. Typical carriers for granular formulations include sand, fuller's earth, bentonite clays, vermiculite, perlite and other organic or inorganic materials which absorb or which may be coated with the toxicant. Granular formulations normally are prepared to contain about 5% to about 25% of active ingredients which may include srface-active agents such heavy aromatic naphthas, kerosene or other petroleum fractions, or vegetable oils; and/or stickers such as destrins, glue or synthetic resins.

Typical wetting, dispersing or emulsifying agents used in agricultural formulations include, for example, the alkyl and alkylaryl sulfonates and sulfastes and their sodium salts; polyhydric alcohols; and other types of surface-active agents, many of which are available in commerce. The surface-active agent, when used, normally comprises from 0.1% to 15% by weight of the herbicidal composition.

Dusts, which are free-flowing admixtures of the active ingredients with finely divided solids such as talc, clays, flours and other organic and inorganic solids which act as dispersants and carriers for the toxicant, are useful formulations for soil-incorporating application.

Pastes, which are homogeneous suspensions of a finely divided solid toxicant in a liquid carrier such as water or oil, are employed for specific purposes. These formulations normally contain about 5% to about 95% of active ingredients by weight, and may also contain small amounts of a wetting, dispersing or emulsifying agent to facilitate dispersion. For application, the pastes are normally diluted and applied as a spray to the area to be affected.

Other useful formulations for herbicidal applications include simple solutions of the active ingredient in a dispersant in which it is completely soluble at the desired concentration, such as acetone, alkylated naphthalenes, xylene and other organic solvents. Pressurized sprays typically aerosols, wherein the active ingredient is dispersed in finely-divided form as a result of vaporization of low boiling dispersant solvent carrier, such as the Freons, may also be used.

The phytotoxic compositions of this invention are applied to the plants in the conventional manner. Thus, the dust and liquid compositions can be applied to the plant by the use of power-dusters, boom and hand sprayers and spray dusters. The compositions can also be applied from airplanes as a dust or spray because they are effective in very low dosages. In order to modify or control growth of germinating seeds or emerging seedlings, as a typical example, the dust and liquid compositions are applied to the soil according to conventional methods and are distributed in the soil to a depth of at least ½ inch below the soil surface. It is not necessary that the phytotoxic compositions be admixed with the soil particles since these compositions can also be applied merely by spraying or sprinkling the surface of the soil. The phytotoxic compositions of this invention can also be applied by addition to irrigation water supplied to the field to be treated. This method of application permits the penetration of the compositions into the soil as the water is absorbed therein. Dust compositions, granular compositions or liquid formulations applied to the surface if the soil can be distributed below the surface of the soil by conventional means such as discing, dragging or mixing operations.

The phytotoxic compositions of this invention can also contain other additaments, for example, fertilizers and other herbicides, pesticides and the like, used as adjuvants or in combination with any of the above described adjuvants. Other phytotoxic compounds useful in combination with the above-described compounds include, for example, anilides such as 2-benzothiazole-2-yloxy-N-methyl acetanilide, 2-chloro-2',6'-dimethyl-N-(n-propylethyl) acetanilide, 2-chloro-2',6'-diethyl-N-butoxymethyl) acetanilide; 2,4-dichlorophenoxyacetic acids, 2,4,5-trichlorophenoxyacetic acid, 2-methyl-4-chlorophenoxyacetic acid and the salts, esters and amides thereof; triazine derivatives, such as 2,4-bis(methoxypropylamino)-6-methylthio-s-triazine, 2-chloro-4-ethylamino-6-isopropylamino-s-triazine, and 2-ethylamino-4-isopropylamino-6-methyl-mercapto-s-triazine; urea derivatives, such as 3-(3,5-dichlorophenyl)-1,1-dimethylurea and 3-(p-chlorophenyl)-1,1-dimethylurea; and acetamides such as N,N-diallyl-α-chloroacetamide, and the like; benzoic acids such as 3-amino-2,5-dichchlorobenzoic acid; thio-carbamates such as S-(1,1-dimethylbenzyl)-piperdene-1-carbothiaote, 3-(4-chlorophenyl)-methyl diethylcarbothioate, ethyl-1-hexahydro-1,4-azepine-1-carbothioate, S-ethyl-hexahydro-1H-azepine-1-carbothioate, S-propyl N,N-dipropylthiocarbamate, S-ethyl N,N-dipropylthiocarbamate, S-ethyl cyclohexylethylthiocarbamate, S-ethyl hexahydro-1H-azepine-1-carbothioate and the like; anilines such as 4-(methylsulfonyl)-2,6-dinitro-N,N-substituted aniline, 4-trifluoromethyl-2,6-dinitro-N,N-di-n-propyl aniline, 4-trifluoromethyl-2,6-dinitro-N-ethyl-N-butyl aniline, 2-[4-(2,4-dichlorophenoxy)phenoxypropanoic acid, 2-[1-(ethoxyimino)-butyl]-5-[2-ethylthio)-propyl]-3-hydroxy-2-cyclohexane-2-cycloxyhexene-1-one, (±)-butyl-2-[4-[5-trifluoromethyl)-2-pyridinyl)-oxy]phenoxy]propanate, sodium 5-[2-chloro-4-(trifluoromethyl)phenoxy]-2-nitrobenzoate, 3-isopropyl-1H-2,1,3-benzothiadiazine-4(3H)-one-2,2-dioxide, and 4-amino-6-tert-butyl-3-(methylthio)-s-triazin-5(4H)-one or (4-amino-6(1,1-dimethylethyl)-3-(methylthio)-1,2,4-triazin-5(4H)-one) and S-(O,O-diisopropyl)-benzene sulfonamide. Fertilizers useful in combination with the active ingredients include, for example, ammonium nitrate, urea and superphosphate. Other useful additaments include materials in which plant organisms take root and grow such as compost, manure, humus, sand, and the like.

**Claims for the Contracting States: BE CH DE FR GB IT LI NL SE**

A compound having the structural formula

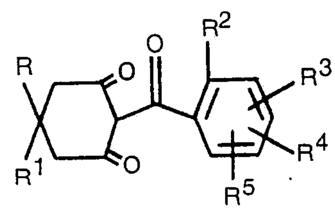

wherein

R and $R^1$ are hydrogen, $C_1$—$C_4$ alkyl, $R^aOC(O)$—, where $R^a$ is $C_1$—$C_4$ alkyl, $R_2$ is chlorine, bromine, iodine or $C_1$—$C_4$ alkoxy, and $R^3$ and $R^4$ and $R^5$ independently are (1) hydrogen, (2) halogen, (3) $C_1$—$C_4$ alkyl, (4) $C_1$—$C_4$ alkoxy, (5) nitro, (6) $C_1$—$C_4$ haloalkyl, (7) $R^bSO_n$— wherein $R^b$ is $C_1$—$C_4$ alkyl and n is the integer 0, 1 or 2, (8)

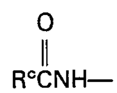

wherein $R^c$ is $C_1$—$C_4$ alkyl, or (9) $R^3$ and $R^4$ together can form a ring structure with two adjacent carbon atoms of the phenyl ring,
and their salts, with the proviso that compounds having the structural formula

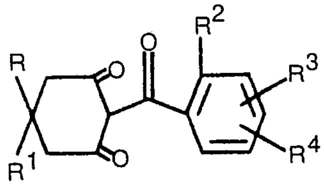

wherein
R and $R^1$ are hydrogen or $C_1$—$C_4$ alkyl,
$R^2$ is chlorine, bromine or iodine,
$R^3$ is hydrogen or halogen, and
$R^4$ is hydrogen, halogen $C_1$—$C_4$ alkyl, $C_1$—$C_4$ alkoxy, nitro, or trifluoromethyl
are excluded.

2. The compounds of Claim 1 wherein R and $R^1$ are hydrogen or $C_1$—$C_4$ alkyl; $R^2$ is chlorine, bromine, iodine or $C_1$—$C_4$ alkoxy; $R^3$ and $R^4$ independently are hydrogen, halogen, $C_1$—$C_4$ alkoxy, nitro, $C_1$—$C_4$ haloalkyl, $R^bSO_n$ wherein $R^b$ is $C_1$—$C_4$ alkyl; and n is the integer 0, 1 or 2; $R^cC(O)NH$— wherein $R^c$ is $C_1$—$C_4$ alkyl; $R^5$ is hydrogen and their salts.

3. The compounds of Claim 1 wherein R and $R^1$ are hydrogen or methyl, $R^2$ is chlorine, bromine or methoxy; $R^3$ is hydrogen, chlorine, dimethylamino or methoxy; $R^4$ is hydrogen, chlorine, nitro, $R^bSO_n$ wherein $R^b$ is $C_1$—$C_4$ alkyl and n is 0 or 2, $SO_2N(CH_3)_2$ or $CF_3$; and $R^5$ is hydrogen, and their salts.

4. The compounds of Claim 3 wherein $R^3$ is substituted at the 3-position, $R^4$ is substituted at the 4-position and $R^5$ is substituted at the 5- or 6-position and their salts.

5. The compounds of Claim 3 wherein R and $R_1$ are hydrogen; $R^2$ is chlorine or methoxy; $R^3$ is hydrogen, 3-chlorine, 3-methoxy, 3-ethoxy, 3-n-propoxy, 4-methoxy or 3-dimethyl-amino and $R^4$ is 4-chlorine, 4-bromine, 4-$CH_3SO_2$—, $4C_2H_5SO_2$—, or 4-$CH_3SO_2$—; and $R^5$ is hydrogen and their salts.

6. The salts of the compounds of Claim 2 wherein $R^2$ is chlorine.

7. The salts of the compounds of Claim 1 wherein R is methyl, $R^1$ is methyl, $R^2$ is chlorine, $R^3$ is hydrogen, $R^4$ is hydrogen and $R^5$ is 6-chlorine.

8. The salts of the compounds of Claim 1 wherein R is hydrogen, $R^1$ is hydrogen, $R^2$ is chlorine, $R^3$ is hydrogen, $R^4$ is hydrogen, $R^5$ is 6-chlorine.

9. The salts of the compounds of Claim 1 wherein R is hydrogen $R^1$ is hydrogen, $R^2$ is chlorine, $R^3$ is hydogen, $R^4$ is 4-chlorine and $R^5$ is hydrogen.

10. The salts of the compounds of Claim 1 wherein R is hydrogen, $R^1$ is hydrogen $R^2$ is chlorine, $R^3$ is 3-chlorine and $R^4$ is hydrogen and $R^5$ is 6-chlorine.

11. The salts of the compounds of Claim 1 wherein R is hydrogen, $R^1$ is hydrogen, $R^2$ is chlorine, $R^3$ is hydrogen, $R^4$ is hydrogen and $R^5$ is 5-trifluoromethyl.

12. The salts of the compounds of Claim 1 wherein R is hydrogen, $R^1$ is hydrogen, $R^2$ is chlorine, $R^3$ is 3-chlorine, $R^4$ is 4-chlorine and $R^5$ is hydrogen.

13. The salts of the compounds of Claim 1 wherein R is hydrogen, $R^1$ is hydrogen, $R^2$ is chlorine, $R^3$ is 3-chlorine, $R^4$ is 4-methoxy and $R^5$ is hydrogen.

14. The salts of the compounds of Claim 1 wherein R is hydrogen, $R^1$ is hydrogen, $R^2$ is chlorine, $R^3$ is hydrogen, $R^4$ is 4-bromine and $R^5$ is hydrogen.

15. The compound of Claim 1 wherein R is hydrogen, $R^1$ is hydrogen, $R^2$ is chlorine, $R^3$ is 3-chlorine, $R^4$ is $nC_3H_7SO_2$— and $R^5$ is hydrogen and its salts.

16. The salts of the compounds of Claim 1 wherein R is hydrogen, $R^1$ is hydrogen, $R^2$ is chlorine, $R^3$ is hydrogen, $R^4$ is 4-chlorine and $R^5$ is hydrogen.

17. The compound of Claim 1 wherein R is isopropyl, $R^1$ is hydrogen, $R^2$ is chlorine, $R^3$ is 3-$CH_3$, $R^4$ is 4-$CH_3SO_2$ and $R^5$ is hydrogen and its salts.

18. The compound of Claim 1 wherein R is hydrogen, $R^1$ is hydrogen, $R^2$ is chlorine, $R^3$ is hydrogen, $R^4$ 4-$CH_3SO_2$ and $R^5$ is hydrogen and its salts.

19. The salts according to Claim 12 namely the sodium, isopropylamine, triethylamine and potassium salts.

20. The method of controlling undesiable vegetation comprising applying to the area where control is desired, a herbicidally effective amount of a compound having the formula

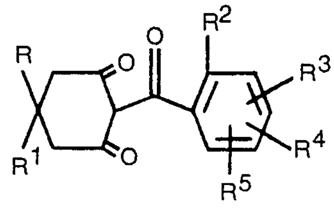

wherein

R and $R^1$ are hydrogen, $C_1$—$C_4$ alkyl, $R^aOC(O)$—, where $R^a$ is $C_1$—$C_4$ alkyl, $R_2$ is chlorine, bromine, iodine or $C_1$—$C_4$ alkoxy, and $R^3$ and $R^4$ and $R^5$ independently are (1) hydrogen, (2) halogen, (3) $C_1$—$C_4$ alkyl, (4) $C_1$—$C_4$ alkoxy, (5) nitro, (6) $C_1$—$C_4$ haloalkyl, (7) $R^bSO_n$— wherein $R^b$ is $C_1$—$C_4$ alkyl and n is the integer 0, 1 or 2, (8)

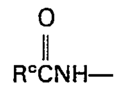

wherein $R^c$ is $C_1$—$C_4$ alkyl, or (9) $R^3$ and $R^4$ together can form a ring structure with two adjacent carbon atoms of the phenyl ring,

or one of its salts with the proviso that compounds having the structural formula

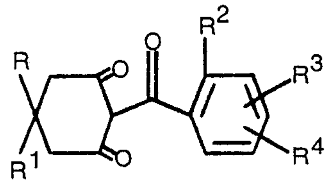

wherein

R and $R^1$ are hydrogen or $C_1$—$C_4$ alkyl,

$R^2$ is chlorine, bromine or iodine,

$R^3$ is hydrogen or halogen, and

$R^4$ is hydrogen, $C_1$—$C_4$ alkyl, $C_1$—$C_4$ alkoxy, nitro, or trifluoromethyl

are excluded.

21. The method of Claim 20 wherein R and $R^1$ are hydrogen or $C_1$—$C_4$ alkyl; $R^2$ is chlorine, bromine, iodine or $C_1$—$C_4$ alkoxy, $R^3$ and $R^4$ independently are hydrogen, halogen, $C_1$—$C_4$ alkyl, $C_1$—$C_4$ alkoxy, nitro, $C_1$—$C_4$ haloalkyl, $R^bSO_n$ wherein $R^b$ is $C_1$—$C_4$ alkyl; and n is the integer 0, 1 or 2; $R^eC(O)NH$— wherein $R^c$ is $C_1$—$C_4$ alkyl; $R^5$ is hydrogen; and their salts.

22. The method of Claim 20 wherein R and $R^1$ are hydrogen or methyl; $R^2$ is chlorine, bromine or methoxy; $R^3$ is hydrogen, chlorine or methoxy; $R^4$ is hydrogen, chlorine nitro, $R^bSO_n$ wherein $R^b$ is $C_1$—$C_4$ alkyl and n is 0 or 2, $SO_2N(CH_3)_2$ or $CF_3$; and $R^5$ is hydrogen and their salts.

23. The method of Claim 22 wherein $R^3$ is substituted at the 3-position, $R^4$ is substituted at the 4-position and $R^5$ is substituted at the 5- or 6-position and their salts.

24. The method of Claim 22 wherein R and $R^1$ are hydrogen, $R^2$ is chlorine or methoxy; $R^3$ is hydrogen, 3-chlorine, 3-methoxy, 3-ethoxy, 3-n-propoxy, 4-methoxy or 3-dimethylamino; $R^4$ is 4-chlorine, 4-bromine, 4-$CH_3SO_2$, 4$C_2H_5SO$— or 4-$CH_3SO_2$; and $R^5$ is hydrogen and their salts.

25. The method of Claim 24 wherein $R^2$ is chlorine and their salts.

26. The method of Claim 20 wherein a salt of a compound wherein R is methyl, $R^1$ is methyl, $R^2$ is chlorine, $R^3$ is hydrogen, and $R^5$ is 6-chlorine is applied.

27. The method of Claim 20 wherein a salt of a compound wherein R is hydrogen, $R^1$ is hydrogen, $R^2$ is chlorine, $R^3$ is hydrogen, $R^4$ is hydrogen, $R^5$ is 6-chlorine is applied.

28. The method of Claim 20 wherein a salt of a compound wherein R is hydrogen, $R^1$ is hydrogen, $R^2$ is chlorine, $R^3$ is hydrogen, $R^4$ is 4-chlorine and $R^5$ is hydrogen is applied.

29. The method of Claim 20 wherein a salt of a compound wherein R is hydrogen, $R^1$ is hydrogen, $R^2$ is chlorine, $R^3$ is 3-chlorine and $R^4$ is hydrogen and $R^5$ is 6-chlorine is applied.

30. The method of Claim 20 wherein a salt of a compound wherein R is hydrogen, $R^1$ is hydrogen, $R^2$ is chlorine, $R^3$ is hydrogen, $R^4$ is hydrogen and $R^5$ is 5-trifluoromethyl is applied.

31. The method of Claim 20 wherein a salt of a compound wherein R is hydrogen, $R^1$ is hydrogen, $R^2$ is chlorine, $R^3$ is 3-chlorine, $R^4$ is 4-chlorine and $R^5$ is hydrogen is applied.

32. The method of Claim 20 wherein a salt of a compound wherein R is hydrogen, $R^1$ is hydrogen, $R^2$ is chlorine, $R^3$ is 3-chlorine, $R^4$ is 4-methoxy and $R^5$ is hydrogen is applied.

33. The method of Claim 20 wherein a salt of a compound wherein R is hydrogen, $R^1$ is hydrogen, $R^2$ is chlorine, $R^3$ is hydrogen, $R^4$ is 4-bromine and $R^5$ is hydrogen is applied.

34. The method of Claim 20 wherein R is hydrogen, $R^1$ is hydrogen, $R^2$ is chlorine, $R^3$ is 3-chlorine, $R^4$ is $nC_3H_7SO_2$— and $R^5$ is hydrogen and its salts.

35. The method of Claim 20 wherein R is isopropyl, $R^1$ is hydrogen, $R^2$ is chlorine, $R^3$ is hydrogen, $R^4$ is 4-chlorine and $R^5$ is hydrogen and its salts.

36. The method of Claim 20 wherein R is hydrogen, $R^1$ is hydrogen, $R^2$ is chlorine, $R^3$ is 3—$CH_3O$, $R^4$ is 4-$CH_3SO_2$, chlorine and $R^5$ is hydrogen and its salts.

37. The method of Claim 20 wherein R is hydrogen, $R^1$ is hydrogen, $R^2$ is chlorine, $R^3$ is hydrogen, $R^4$ is 4-$CH_3SO_2$ and $R^5$ is hydrogen and its salts.

38. The method of Claim 20 wherein the salts are the sodium, isopropylamine, triethylamine and potassium salts.

39. The compound of Claim 1 wherein R is hydrogen, $R^1$ is hydrogen, $R^2$ is chlorine, $R^3$ is 3—$C_2H_5S$, $R^4$ is 4—$C_2H_5SO_2$ and $R^5$ is hydrogen and its salts.

40. The compound of Claim 1 wherein R is hydrogen, $R^1$ is hydrogen, $R^2$ is chlorine, $R^3$ is 3-methoxy, $R^4$ is 4—$C_2H_5SO_2$ and $R^5$ is hydrogen and its salts.

41. The compound of Claim 1 wherein R is hydrogen, $R^1$ is hydrogen, $R^2$ is chlorine, $R^3$ is 3-chlorine, $R^4$ is 4-n-$C_3H_7SO_2$— and $R^5$ is hydrogen and its salts.

42. The method of Claim 20 wherein R is hydrogen, $R^1$ is hydrogen, $R^2$ is chlorine, $R^3$ is 3—$C_2H_5S$, $R^4$ is 4-$C_3H_7SO_2$— and $R^5$ is hydrogen and its salts.

43. The method of Claim 20 wherein R is hydrogen, $R^1$ is hydrogen, $R^2$ is chlorine, $R^3$ is 3-methoxy, $R^4$ is 4-$CH_3SO_2$ and $R^5$ is hydrogen and its salts.

44. The method of Claim 20 wherein R is hydrogen, $R^1$ is hydrogen, $R^2$ is chlorine, $R^3$ is 3-chlorine, $R^4$ is 4-n-$C_3H_7SO_2$— and $R^5$ is hydrogen and its salts.

45 A herbicidal composition comprising the compound of Claim 18 or one of its salts and an inert carrier therefor.

46. The method of controlling undesirable vegetation comprising applying to the area where control is desired a herbicidally effective amount of the compound of Claim 18 or one of its salts and an inert carrier therefor.

**Claims for the Contracting State: AT**

A process for preparing compounds having the structural formula

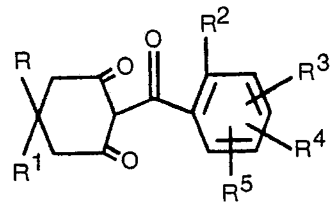

wherein

R and $R^1$ are hydrogen, $C_1$—$C_4$ alkyl, $R^aOC(O)$—, where $R^a$ is $C_1$—$C_4$ alkyl, $R_2$ is chlorine, bromine, iodine or $C_1$—$C_4$ alkoxy, and $R^3$ and $R^4$ and $R^5$ independently are (1) hydrogen, (2) halogen, (3) $C_1$—$C_4$ alkyl, (4) $C_1$—$C_4$ alkoxy, (5) nitro, (6) $C_1$—$C_4$ haloalkyl, (7) $R^bSO_n$— wherein $R^b$ is $C_1$—$C_4$ alkyl and n is the integer 0, 1 or 2, (8)

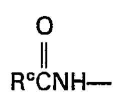

wherein $R^c$ is $C_1$—$C_4$ alkyl, or (9) $R^3$ and $R^4$ together can form a ring structure with two adjacent carbon atoms of the phenyl ring,

and their salts, with the proviso that compounds having the structural formula

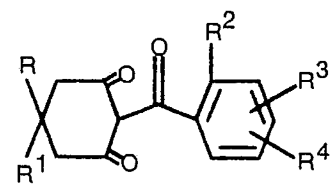

wherein

R and $R^1$ are hydrogen or $C_1$—$C_4$ alkyl,

$R^2$ is chlorine, bromine or iodine,

$R^3$ is hydrogen or halogen, and

$R^4$ is hydrogen, $C_1$—$C_4$ alkyl, $C_1$—$C_4$ alkoxy, nitro, or trifluoromethyl

are excluded.

comprising reacting a substituted dione of the general formula II

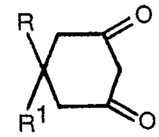

with a substituted benzoyl cyanide of the general formula III

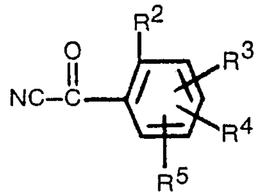

and working up the reaction product by conventional techniques.

2. The process of Claim 1 wherein R and $R^1$ are hydrogen or $C_1$—$C_4$ alkyl; $R^2$ is chlorine, bromine, iodine or $C_1$—$C_4$ alkoxy; $R^3$ and $R^4$ independently are hydrogen, halogen, $C_1$—$C_4$ alkoxy, nitro, $C_1$—$C_4$ haloalkyl, $R^bSO_n$ wherein $R^b$ is $C_1$—$C_4$ alkyl; and n is the integer 0, 1 or 2; $R^cC(O)NH$— wherein is $C_1$—$C_4$ alkyl; $R^5$ is hydrogen and their salts.

3. The process of Claim 1 wherein R and $R^1$ are hydrogen or methyl, $R^2$ is chlorine, bromine or methoxy; $R^3$ is hydrogen, chlorine, dimethylamino or methoxy; $R^4$ is hydrogen, chlorine, nitro, $R^bSO_n$ wherein $R^b$ is $C_1$—$C_4$ alkyl and n is 0 or 2, $SO_2N(CH_3)_2$ or $CF_3$; and $R^5$ is hydrogen, and their salts.

4. The process of Claim 3 wherein $R^3$ is substituted at the 3-position, $R^4$ is substituted at the 4-position and $R^5$ their salts.

5. The process of Claim 3 wherein R and $R^1$ are hydrogen; $R^2$ is chlorine or methoxy; $R^3$ is hydrogen, 3-chlorine, 3-methoxy, 3-ethoxy, 3-n-propoxy, 4-methoxy or 3-dimethyl-amino and $R^4$ is 4-chlorine, 4-bromine, 4-$CH_3SO_2$—, $4C_2H_5SO_2$—, or 4-$CF_3SO_2$—; and $R^5$ is hydrogen and their salts.

6. The process of Claim 2 wherein $R^2$ is chlorine are prepared.

7. The process of Claim 1 wherein salts of the compounds wherein R is methyl, $R^1$ is methyl, $R^2$ is chlorine, $R^3$ is hydrogen, $R^4$ is hydrogen and $R^5$ is 6-chlorine are prepared.

8. The process of Claim 1 wherein salts of the compound wherein R is hydrogen, $R^1$ is hydrogen, $R^2$ is chlorine, $R^3$ is hydrogen, $R^4$ is hydrogen, $R^5$ is 6-chlorine are prepared.

9. The process of Claim 1 wherein salts of the compound wherein R is hydrogen $R^1$ is hydrogen, $R^2$ is chlorine, $R^3$ is hydrogen, $R^4$ is 4-chlorine and $R^5$ is hydrogen are prepared.

10. The process of Claim 1 wherein salts of the compound wherein R is hydrogen, $R^1$ is hydrogen $R^2$ is chlorine, $R^3$ is 3-chlorine and $R^4$ is hydrogen and $R^5$ is 6-chlorine are prepared.

11. The process of Claim 1 wherein salts of the compound wherein R is hydrogen, $R^1$ is hydrogen, $R^2$ is chlorine, $R^3$ is hydrogen, $R^4$ is hydrogen and $R^5$ is 5-trifluoromethyl are prepared.

12. The process of Claim 1 wherein salts of the compound wherein R is hydrogen, $R^1$ is hydrogen, $R^2$ is chlorine, $R^3$ is 3-chlorine, $R^4$ is 4-chlorine and $R^5$ is hydrogen are prepared.

13. The process of Claim 1 wherein salts of the compound wherein R is hydrogen, $R^1$ is hydrogen, $R^2$ is chlorine, $R^3$ is 3-chlorine, $R^4$ is 4-methoxy and $R^5$ is hydrogen are prepared.

14. The process of Claim 1 wherein salts of the compound wherein R is hydrogen, $R^1$ is hydrogen, $R^2$ is chlorine, $R^3$ is hydrogen, $R^4$ is 4-bromine and $R^5$ is hydrogen are prepared.

15. The process of Claim 1 wherein R is hydrogen, $R^1$ is hydrogen, $R^2$ is chlorine, $R^3$ is 3-chlorine, $R^4$ is $nC_3H_7SO_2$— and $R^5$ is hydrogen and its salts.

16. The process of Claim 1 wherein salts of the compound wherein R is hydrogen, $R^1$ is hydrogen, $R^2$ is chlorine, $R^3$ is hydrogen, $R^4$ is 4-chlorine and $R^5$ is hydrogen are prepared.

17. The process of Claim 1 wherein R is isopropyl, $R^1$ is hydrogen, $R^2$ is chlorine, $R^3$ is 3-$CH_3$, $R^4$ 4-$CH_3SO_2$ and $R^5$ is hydrogen and its salts.

18. The process of Claim 1 wherein R is hydrogen, $R^1$ is hydrogen, $R^2$ is chlorine, $R^3$ is hydrogen, $R^4$ 4-$CH_3SO_2$ and $R^5$ is hydrogen and its salts.

19. The process according to claim 12 wherein the sodium, isopropylamine, triethylamine and potassium salts are prepared.

20. The method of controlling undesirable vegetation comprising applying to the area where control is desired, a herbicidally effective amount of a compound having the formula

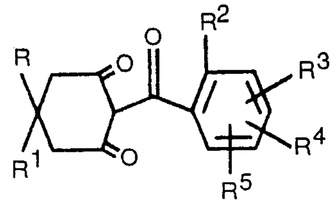

wherein

R and $R^1$ are hydrogen, $C_1$—$C_4$ alkyl, $R^aOC(O)$—, where $R^a$ is $C_1$—$C_4$ alkyl, $R_2$ is chlorine, bromine, iodine or $C_1$—$C_4$ alkoxy, and $R^3$ and $R^4$ and $R^5$ independently are (1) hydrogen, (2) halogen, (3) $C_1$—$C_4$ alkyl, (4) $C_1$—$C_4$ alkoxy, (5) nitro, (6) $C_1$—$C_4$ haloalkyl, (7) $R^bSO_n$— wherein $R^b$ is $C_1$—$C_4$ alkyl and n is the integer 0, 1 or 2, (8)

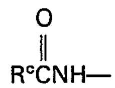

wherein $R^c$ is $C_1$—$C_4$ alkyl, or (9) $R^3$ and $R^4$ together can form a ring structure with two adjacent carbon atoms of the phenyl ring,
or one of its salts with the proviso that compounds having the structural formula

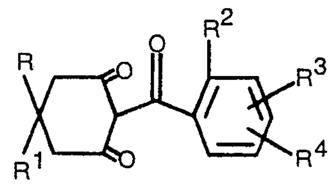

wherein
R and $R^1$ are hydrogen or $C_1$—$C_4$ alkyl,
$R^2$ is chlorine, bromine or iodine,
$R^3$ is hydrogen or halogen, and
$R^4$ is hydrogen, $C_1$—$C_4$ alkyl, $C_1$—$C_4$ alkoxy, nitro, or trifluoromethyl
are excluded.

21. The method of claim 20 wherein R and $R^1$ are hydrogen or $C_1$—$C_4$ alkyl; $R^2$ is chlorine, bromine, iodine or $C_1$—$C_4$ alkoxy, $R^3$ and $R^4$ independently are hydrogen, halogen, $C_1$—$C_4$ alkyl, $C_1$—$C_4$ alkoxy, nitro, $C_1$—$C_4$ haloalkyl, $R^bSO_n$ wherein $R^b$ is $C_1$—$C_4$ alkyl; and n is the integer 0, 1 or 2; $R^eC(O)NH$— wherein $R^c$ is $C_1$—$C_4$ alkyl; $R^5$ is hydrogen; and their salts.

22. The method of claim 22 wherein R and $R^1$ are hydrogen or methyl; $R^2$ is chlorine, bromine or methoxy; $R^3$ is hydrogen, chlorine or methoxy; $R^4$ is hydrogen, chlorine nitro, $R^bSO_n$ wherein $R^b$ is $C_1$—$C_4$ alkyl and n is 0 or 2, $SO_2N(CH_3)_2$ or $CF_3$; and $R^5$ is hydrogen and their salts.

23. The method of Claim 22 wherein $R^3$ is substituted at the 3-position, $R^4$ is substituted at the 4-position and $R^5$ is substituted at the 5- or 6-position and their salts.

24. The method of Claim 22 wherein R and $R^1$ are hydrogen, $R^2$ is chlorine or methoxy; $R^3$ is hydrogen, 3-chlorine, 3-methoxy, 3-ethoxy, 3-n-propoxy, 4-methoxy or 3-dimethylamino; $R^4$ is 4-chlorine, 4-bromine, 4-$CH_3SO_2$, $4C_2H_5SO_2$— or 4-$CH_3SO_2$; and $R^5$ is hydrogen and their salts.

25. The method of Claim 24 wherein $R^2$ is chlorine and their salts.

26. The method of Claim 20 wherein a salt of a compound wherein R is methyl, $R^1$ is methyl, $R^2$ is chlorine, $R^3$ is hydrogen, $R^4$ is hydrogen and $R^5$ is 6-chlorine is applied.

27. The method of Claim 20 wherein a salt of a compound wherein R is hydrogen, $R^1$ is hydrogen, $R^2$ is chlorine, $R^3$ is hydrogen, $R^4$ is hydrogen, $R^5$ is 6-chlorine is applied.

28. The method of Claim 20 wherein a salt of a compound wherein R is hydrogen, $R^1$ is hydrogen, $R^2$ is chlorine, $R^3$ is hydrogen, $R^4$ is 4-chlorine and $R^5$ is hydrogen is applied.

29. The method of Claim 20 wherein a salt of a compound wherein R is hydrogen, $R^1$ is hydrogen, $R^2$ is chlorine, $R^3$ is 3-chlorine and $R^4$ is hydrogen and $R^5$ is 6-chlorine is applied.

30. The method of Claim 20 wherein a salt of a compound wherein R is hydrogen, $R^1$ is hydrogen, $R^2$ is chlorine, $R^3$ is hydrogen, $R^4$ is hydrogen and $R^5$ is 5-trifluoromethyl is applied.

31. The method of Claim 20 wherein a salt of a compound wherein R is hydrogen, $R^1$ is hydrogen, $R^2$ is chlorine, $R^3$ is 3-chlorine, $R^4$ is 4-chlorine and $R^5$ is hydrogen is applied.

32. The method of Claim 20 wherein a salt of a compound wherein R is hydrogen, $R^1$ is hydrogen, $R^2$ is chlorine, $R^3$ is 3-chlorine, $R^4$ is 4-methoxy and $R^5$ is hydrogen is applied.

33. The method of Claim 20 wherein a salt of a compound wherein R is hydrogen, $R^1$ is hydrogen, $R^2$ is chlorine, $R^3$ is hydrogen, $R^4$ is 4-bromine and $R^5$ is hydrogen is applied.

34. The method of Claim 20 wherein R is hydrogen, $R^1$ is hydrogen, $R^2$ is chlorine, $R^3$ is 3-chlorine, $R^4$ is $nC_3H_7SO_2$— and $R^5$ is hydrogen and its salts.

35. The method of Claim 20 wherein R is isopropyl, $R^1$ is hydrogen, $R^2$ is chlorine, $R^3$ is hydrogen, $R^4$ is 4-chlorine and $R^5$ is hydrogen and its salts.

36. The method of Claim 20 wherein R is hydrogen, $R^1_a$ is hydrogen, $R^2$ is chlorine, $R^3$ is 3—$CH_3O$, $R^4$ is 4-$CH_3SO_2$, chlorine and $R^5$ is hydrogen and its salts.

37. The method of Claim 20 wherein R is hydrogen, $R^1$ is hydrogen, $R^2$ is chlorine, $R^3$ is hydrogen, $R^4$ is 4-$CH_3SO_2$ and $R^5$ is hydrogen and its salts.

38. The method of Claim 20 wherein the salts are the sodium, isopropylamine, triethylamine and potassium salts.

39. The process of Claim 1 wherein R is hydrogen, $R^1$ is hydrogen, $R^2$ is chlorine, $R^3$ is 3—$C_2H_5S$, $R^4$ is 4—$C_2H_5SO_2$ and $R^5$ is hydrogen and its salts.

40. The process of Claim 1 wherein R is hydrogen, $R^1$ is hydrogen, $R^2$ is chlorine, $R^3$ is 3-methoxy, $R^4$ is 4—$CH_3SO_2$ and $R^5$ is hydrogen and its salts.

41. The process of Claim 1 wherein R is hydrogen, $R^1$ is hydrogen, $R^2$ is chlorine, $R^3$ is 3-chlorine, $R^4$ is 4-n-$C_3H_7SO_2$— and $R^5$ is hydrogen and its salts.

42. The method of Claim 20 wherein R is hydrogen, $R^1$ is hydrogen, $R^2$ is chlorine, $R^3$ is 3—$C_2H_5S$, $R^4$ is 4-$C_2H_5SO_2$— and $R^5$ is hydrogen and its salts.

43. The method of Claim 20 wherein R is hydrogen, $R^1$ is hydrogen, $R^2$ is chlorine, $R^3$ is 3-methoxy, $R^4$ is 4-$CH_3SO_2$ and $R^5$ is hydrogen and its salts.

44. The method of Claim 20 wherein R is hydrogen, $R^1$ is hydrogen, $R^2$ is chlorine, $R^3$ is 3-chlorine, $R^4$ is 4-n-$C_3H_7SO_2$— and $R^5$ is hydrogen and its salts.

45. A process for preparing a herbicidal composition comprising mixing the compound of Claim 18 or one of its salts and an inert carrier therefor.

46. The method of controlling undesirable vegetation comprising applying to the area where control is desired, a herbicidally effective amount of the compound of Claim 18 or one of its salts and an inert carrier therefor.

**Patentansprüche für die Vertragsstaaten: BE GB FR DE NL IT SE CH LI**

1. Verbindung der Strukturformel

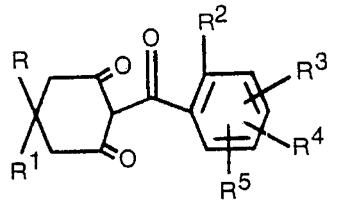

dadurch gekennzeichnet, daß

R und $R^1$ Wasserstoff, $C_1$—$C_4$-Alkyl, $R^aOC(O)$—, worin $R^a$ für $C_1$—$C_4$-Alkyl steht, bedeuten,

$R^2$ Chlor, Brom, Jod oder $C_1$—$C_4$-Alkoxy bedeutet, und

$R^3$ und $R^4$ und $R^5$ unabhängig (1) Wasserstoff, (2) Halogen, (3) $C_1$—$C_4$-Alkyl, (4) $C_1$—$C_4$-Alkoxy, (5) Nitro (6) $C_1$—$C_4$-Haloalkyl, (7) $R^bSO_n$—, worin $R^b$ für $C_1$—$C_4$-Alkyl steht und n eine ganze Zahl von 0, 1 oder 2 bedeutet, (8)

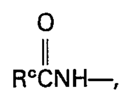

worin $R^c$ für $C_1$—$C_4$-Alkyl steht, bedeuten, (9) $R^3$ und $R^4$ zusammen mit zwei benachbarten Kohlenstoffatomen des Phenylrings eine Ringstruktur bilden,

und ihre Salze, mit der Maßgabe, daß Verbindungen der Strukturformel

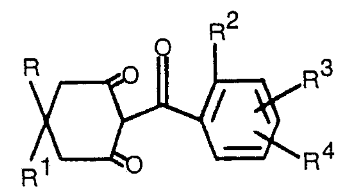

worin

R und $R^1$ Wasserstoff oder $C_1$—$C_4$-Alkyl,

$R^2$ Chlor, Brom oder Jod,

$R^3$ Wasserstoff oder Halogen, und

$R^4$ Wasserstoff, Halogen, $C_1$—$C_4$-Alkyl, $C_1$—$C_4$-Alkoxy, Nitro oder Trifluormethyl bedeuten,

ausgeschlossen sind.

2. Verbindung nach Anspruch 1, dadurch gekennzeichnet, daß R und $R^1$ Wasserstoff oder $C_1$—$C_4$-Alkyl; $R^2$ Chlor, Brom, Jod oder $C_1$—$C_4$-Alkoxy; $R^3$ und $R^4$ unabhängig Wasserstoff, Halogen, $C_1$—$C_4$-Alkyl, $C_1$—$C_4$-Alkoxy, Nitro, $C_1$—$C_4$-Haloalkyl, $R^bSO_n$, worin $R^b$ für $C_1$—$C_4$-Alkyl steht und n die ganze Zahl 0, 1 oder 2 bedeutet; $R^cC(O)NH$—, worin $R^c$ für $C_1$—$C_4$-Alkyl steht; $R^5$ Wasserstoff bedeuten, und ihre Salze.

3. Verbindungen nach Anspruch 1, dadurch gekennzeichnet, daß R und $R^1$ Wasserstoff oder Methyl; $R^2$ Chlor, Brom oder Methoxy; $R^3$ Wasserstoff, Chlor, Dimethylamino oder Methoxy; $R^4$ Wasserstoff, Chlor, Nitro, $R^bSO_n$, worin $R^b$ für $C_1$—$C_4$-Alkyl steht und n 0 oder 2 bedeutet, $SO_2N(CH_3)_3$ oder $CF_3$; und $R^5$ Wasserstoff bedeuten, und ihre Salze.

4. Verbindungen nach Anspruch 3, dadurch gekennzeichnet, daß $R^3$ in der 3-Stellung substituiert ist, $R^4$ in der 4-Stellung substituiert ist und $R^5$ in der 5- oder 6-Stellung substituiert ist, und ihre Salze.

5. Verbindungen nach Anspruch 3, dadurch gekennzeichnet, daß R und $R^1$ Wasserstoff; $R^2$ Chlor oder Methoxy; $R^3$ Wasserstoff, 3-Chlor, 3-Methoxy, 3-Ethoxy, 3-n-Propoxy, 4-Methoxy oder 3-Dimethylamino

und $R^4$ 4-Chlor, 4-Brom, 4-$CH_3SO_2$—, 4$C_2H_5SO_2$— oder 4-$CF_3SO_2$ und $R^5$ Wasserstoff bedeuten, und ihre Salze.

6. Salze der Verbindungen nach Anspruch 2, dadurch gekennzeichnet, daß $R^2$ Chlor bedeutet.

7. Salze der Verbindungen nach Anspruch 1, dadurch gekennzeichnet, daß R Methyl, $R^1$ Methyl, $R^2$ Chlor, $R^3$ Wasserstoff, $R^4$ Wasserstoff und $R^5$ 6-Chlor bedeuten.

8. Salze der Verbindung nach Anspruch 1, dadurch gekennzeichnet, daß R Wasserstoff, $R^1$ Wasserstoff, $R^2$ Chlor, $R^3$ Wasserstoff, $R^4$ Wasserstoff, $R^5$ 6-Chlor bedeuten.

9. Salze der Verbindung nach Anspruch 1, dadurch gekennzeichnet, daß R Wasserstoff, $R^1$ Wasserstoff, $R^2$ Chlor, $R^3$ Wasserstoff, $R^4$ 4-Chlor und $R^5$ Wasserstoff bedeuten.

10. Salze der Verbindung nach Anspruch 1, dadurch gekennzeichnet, daß R Wasserstoff, $R^1$ Wasserstoff, $R^2$ Chlor, $R^3$ 3-Chlor und $R^4$ Wasserstoff und $R^5$ 6-Chlor bedeuten.

11. Salze der Verbindung nach Anspruch 1, dadurch gekennzeichnet, daß R Wasserstoff, $R^1$ Wasserstoff, $R^2$ Chlor, $R^3$ Wasserstoff, $R^4$ Wasserstoff und $R^5$ 5-Trifluormethyl bedeuten.

12. Salze der Verbindung nach Anspruch 1, dadurch gekennzeichnet, daß R Wasserstoff, $R^1$ Wasserstoff, $R^2$ Chlor, $R^3$ 3-Chlor, $R^4$ 4-Chlor und $R^5$ Wasserstoff bedeuten.

13. Salze der Verbindung nach Anspruch 1, dadurch gekennzeichnet, daß R Wasserstoff, $R^1$ Wasserstoff, $R^2$ Chlor, $R^3$ 3-Chlor, $R^4$ 4-Methoxy und $R^5$ Wasserstoff bedeuten.

14. Salze der Verbindung nach Anspruch 1, dadurch gekennzeichnet, daß R Wasserstoff, $R^1$ Wasserstoff, $R^2$ Chlor, $R^3$ Wasserstoff, $R^4$ 4-Brom und $R^5$ Wasserstoff bedeuten.

15. Verbindung nach Anspruch 1, dadurch gekennzeichnet, daß R Wasserstoff, $R^1$ Wasserstoff, $R^2$ Chlor, $R^3$ 3-Chlor, $R^4$ $nC_3H_7SO_2$— und $R^5$ Wasserstoff bedeuten, und ihre Salze.

16. Salze der Verbindung nach Anspruch 1, dadurch gekennzeichnet, daß R Isopropyl, $R^1$ Wasserstoff, $R^2$ Chlor, $R^3$ Wasserstoff, $R^4$ 4-Chlor und $R^5$ Wasserstoff bedeuten.

17. Verbindung nach Anspruch 1, dadurch gekennzeichnet, daß R Isopropyl, $R^1$ Wasserstoff, $R^2$ Chlor, $R^3$ 3-$CH_3$, $R^4$ 4-$CH_3SO_2$ und $R^5$ Wasserstoff bedeuten, und ihre Salze.

18. Verbindung nach Anspruch 1, dadurch gekennzeichnet, daß R Wasserstoff, $R^1$ Wasserstoff, $R^2$ Chlor, $R^3$ Wasserstoff, $R^4$ 4-$CH_3SO_2$ und $R^5$ Wasserstoff bedeuten, und ihre Salze.

19. Salze nach Anspruch 12, nämlich Natrium-, Isopropylamin, Triethylamin- und Kaliumsalze.

20. Verfahren zur Kontrolle unerwünschter Vegetation, dadurch gekennzeichnet, daß man auf die Fläche, wo eine Kontrolle erfolgen soll, eine herbizid wirksame Menge der Formel

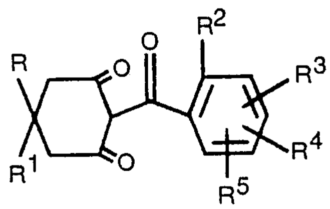

worin

R und $R^1$ Wasserstoff, $C_1$—$C_4$-Alkyl, $R^aOC(O)$—, worin $R^a$ für $C_1$—$C_4$-Alkyl steht, bedeuten,

$R^2$ Chlor, Brom, Jod oder $C_1$—$C_4$-Alkoxy bedeutet, und

$R^3$ und $R^4$ und $R^5$ unabhängig (1) Wasserstoff, (2) Halogen, (3) $C_1$—$C_4$-Alkyl, (4) $C_1$—$C_4$-Alkoxy, (5) Nitro, (6) $C_1$—$C_4$-Haloalkyl, (7) $R^bSO_n$—, worin $R^b$ für $C_1$—$C_4$-Alkyl steht und n eine ganze Zahl von 0, 1 oder 2 bedeutet, (8)

$$\overset{O}{\overset{\|}{R^cC}}NH—,$$

worin $R^c$ für $C_1$—$C_4$-Alkyl steht, bedeuten, (9) $R^3$ und $R^4$ zusammen mit zwei benachbarten Kohlenstoffatomen des Phenylrings eine Ringstruktur bilden,

oder eines ihrer Salze anwendet, mit der Maßgabe, daß Verbindungen der Strukturformel

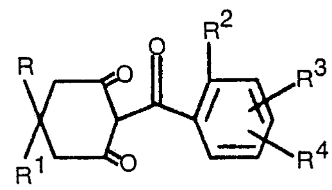

worin

R und $R^1$ Wasserstoff oder $C_1$—$C_4$-Alkyl,

$R^2$ Chlor, Brom oder Jod,

$R^3$ Wasserstoff oder Halogen und

$R^4$ Wasserstoff, Halogen, $C_1$—$C_4$-Alkyl, $C_1$—$C_4$-Alkoxy, Nitro oder Trifluormethyl bedeuten, ausgeschlossen sind.

21. Verfahren nach Anspruch 20, dadurch gekennzeichnet, daß R und $R^1$ Wasserstoff oder $C_1$—$C_4$-Alkyl; $R^2$ Chlor, Brom, Jod oder $C_1$—$C_4$-Alkoxy; $R^3$ und $R^4$ unabhängig Wasserstoff, Halogen, $C_1$—$C_4$-Alkyl, $C_1$—$C_4$-Alkoxy, Nitro, $C_1$—$C_4$-Haloalkyl, $R^bSO_n$, worin $R^b$ für $C_1$—$C_4$-Alkyl steht und n die ganze Zahl 0, 1 oder 2 bedeutet; $R^cC(O)NH$—, worin $R^c$ für $C_1$—$C_4$-Alkyl steht; $R^5$ Wasserstoff bedeuten, und ihre Salze.

22. Verfahren nach Anspruch 20, dadurch gekennzeichnet, daß R und $R^1$ Wasserstoff oder Methyl; $R^2$ Chlor, Brom oder Methoxy; $R^3$ Wasserstoff, Chlor, Methoxy; $R^4$ Wasserstoff, Chlor, Nitro, $R^bSO_n$, worin $R^b$ für $C_1$—$C_4$-Alkyl steht und n 0 oder 2 bedeutet, $SO_2N(CH_3)_2$ oder $CF_3$; und $R^5$ Wasserstoff bedeuten, und ihre Salze.

23. Verfahren nach Anspruch 22, dadurch gekennzeichnet, daß $R^3$ in der 3-Stellung substituiert ist, $R^4$ in der 4-Stellung substituiert ist und $R^5$ in der 5- oder 6-Stellung substituiert ist, und ihre Salze.

24. Verfahren nach Anspruch 22, dadurch gekennzeichnet, daß R und $R^1$ Wasserstoff; $R^2$ Chlor oder Methoxy; $R^3$ Wasserstoff, 3-Chlor, 3-Methoxy, 3-Ethoxy, 3-n-Propoxy, 4-Methoxy oder 3-Dimethylamino und $R^4$ 4-Chlor, 4-Brom, 4-$CH_3SO_2$—, $4C_2H_5SO_2$— oder 4-$CF_3SO_2$ und $R^5$ Wasserstoff bedeuten, und ihre Salze.

25. Verfahren nach Anspruch 24, dadurch gekennzeichnet, daß $R^2$ Chlor bedeutet, und ihre Salze.

26. Verfahren nach Anspruch 20, dadurch gekennzeichnet, daß ein Salz einer Verbindung, worin R Methyl, $R^1$ Methyl, $R^2$ Chlor, $R^3$ Wasserstoff, $R^4$ Wasserstoff und $R^5$ 6-Chlor bedeuten, angewendet wird.

27. Verfahren nach Anspruch 20, dadurch gekennzeichnet, daß ein Salz einer Verbindung, worin R Wasserstoff, $R^1$ Wasserstoff, $R^2$ Chlor, $R^3$ Wasserstoff, $R^4$ Wasserstoff, $R^5$ 6-Chlor bedeuten, angewendet wird.

28. Verfahren nach Anspruch 20, dadurch gekennzeichnet, daß ein Salz einer Verbindung, worin R Wasserstoff, $R^1$ Wasserstoff, $R^2$ Chlor, $R^3$ Wasserstoff, $R^4$ 4-Chlor und $R^5$ Wasserstoff bedeuten, angewendet wird.

29. Verfahren nach Anspruch 20, dadurch gekennzeichnet, daß ein Salz einer Verbindung, worin R Wasserstoff, $R^1$ Wasserstoff, $R^2$ Chlor, $R^3$ 3-Chlor und $R^4$ Wasserstoff und $R^5$ 6-Chlor bedeuten, angewendet wird.

30. Verfahren nach Anspruch 20, dadurch gekennzeichnet, daß ein Salz einer Verbindung, worin R Wasserstoff, $R^1$ Wasserstoff, $R^2$ Chlor, $R^3$ Wasserstoff, $R^4$ Wasserstoff und $R^5$ 5-Trifluormethyl bedeuten, angewendet wird.

31. Verfahren nach Anspruch 20, dadurch gekennzeichnet, daß ein Salz einer Verbindung, worin R Wasserstoff, $R^1$ Wasserstoff, $R^2$ Chlor, $R^3$ 3-Chlor, $R^4$ 4-Chlor und $R^5$ Wasserstoff bedeuten, angewendet wird.

32. Verfahren nach Anspruch 20, dadurch gekennzeichnet, daß ein Salz einer Verbindung, worin R Wasserstoff, $R^1$ Wasserstoff, $R^2$ Chlor, $R^3$ 3-Chlor, $R^4$ 4-Methoxy und $R^5$ Wasserstoff bedeuten, angewendet wird.

33. Verfahren nach Anspruch 20, dadurch gekennzeichnet, daß ein Salz einer Verbindung, worin R Wasserstoff, $R^1$ Wasserstoff, $R^2$ Chlor, $R^3$ Wasserstoff, $R^4$ 4-Brom und $R^5$ Wasserstoff bedeuten, angewendet wird.

34. Verfahren nach Anspruch 20, dadurch gekennzeichnet, daß R Wasserstoff, $R^1$ Wasserstoff, $R^2$ Chlor, $R^3$ 3-Chlor, $R^4$ $nC_3H_7SO_2$— und $R^5$ Wasserstoff bedeuten, und ihre Salze.

35. Verfahren nach Anspruch 20, dadurch gekennzeichnet, daß R Isopropyl, $R^1$ Wasserstoff, $R^2$ Chlor, $R^3$ Wasserstoff, $R^4$ 4-Chlor und $R^5$ Wasserstoff bedeuten, und ihre Salze.

36. Verfahren nach Anspruch 20, dadurch gekennzeichnet, daß R Wasserstoff, $R^1$ Wasserstoff, $R^2$ Chlor, $R^3$ 3-$CH_3O$, $R^4$ 4-$CH_3SO_2$, Chlor und $R^5$ Wasserstoff bedeuten, und ihre Salze.

37. Verfahren nach Anspruch 20, dadurch gekennzeichnet, daß R Wasserstoff, $R^1$ Wasserstoff, $R^2$ Chlor, $R^3$ Wasserstoff, $R^4$ 4-$CH_3SO_2$ und $R^5$ Wasserstoff bedeuten, und ihre Salze.

38. Verfahren nach Anspruch 20, dadurch gekennzeichnet, daß die Salze die Natrium-, Isopropylamin-, Triethylamin- und Kaliumsalze sind.

39. Verfahren nach Anspruch 1, dadurch gekennzeichnet, daß R Wasserstoff, $R^1$ Wasserstoff, $R^2$ Chlor, $R^3$ 3-$C_2H_5S$, $R^4$ 4-$C_2H_5SO_2$ und $R_5$ Wasserstoff bedeuten, und ihre Salze.

40. Verfahren nach Anspruch 1, dadurch gekennzeichnet, daß R Wasserstoff, $R^1$ Wasserstoff, $R^2$ Chlor, $R^3$ 3-Methoxy, $R^4$ 4-$CH_3SO_2$ und $R^5$ Wasserstoff bedeuten, und ihre Salze.

41. Verfahren nach Anspruch 1, dadurch gekennzeichnet, daß R Wasserstoff, $R^1$ Wasserstoff, $R^2$ Chlor, $R^3$ 3-Chlor, $R^4$ 4-n-$C_3H_7SO_2$— und $R^5$ Wasserstoff bedeuten, und ihre Salze.

42. Verfahren nach Anspruch 20, dadurch gekennzeichnet, daß R Wasserstoff, $R^1$ Wasserstoff, $R^2$ Chlor, $R^3$ 3-$C_2H_5S$, $R^4$ 4-$C_2H_5SO_2$ und $R^5$ Wasserstoff bedeuten, und ihre Salze.

43. Verfahren nach Anspruch 20, dadurch gekennzeichnet, daß R Wasserstoff, $R^1$ Wasserstoff, $R^2$ Chlor, $R^3$ 3-Methoxy, $R^4$ 4-$CH_3SO_2$ und $R^5$ Wasserstoff bedeuten, und ihre Salze.

44. Verfahren nach Anspruch 20, dadurch gekennzeichnet, daß R Wasserstoff, $R^1$ Wasserstoff, $R^2$ Chlor, $R^3$ 3-Chlor, $R^4$ 4-n-$C_3H_7SO_2$— und $R^5$ Wasserstoff bedeuten, und ihre Salze.

45. Herbizide Zubereitung, dadurch gekennzeichnet, daß sie die Verbindung nach Anspruch 18 oder eines ihrer Salze und einen inerten Träger dafür enthält.

46. Verfahren zur Kontrolle unerwünschter Vegetation, dadurch gekennzeichnet, daß man auf die

Fläche, wo eine Kontrolle erfolgen soll, eine herbizid wirksame Menge der Verbindung nach Anspruch 18 oder eines ihrer Salze und einen inerten Träger dafür anwendet.

**Patentansprüche für den Vertragsstaat: AT**

1. Verfahren zur Herstellung von Verbindungen der Strukturformel

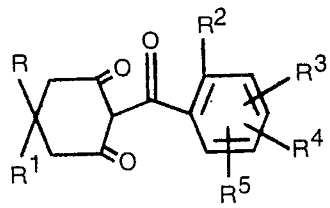

worin

R und $R^1$ Wasserstoff, $C_1$—$C_4$-Alkyl, $R^aOC(O)$—, worin $R^a$ für $C_1$—$C_4$-Alkyl steht, bedeuten,

$R^2$ Chlor, Brom, Jod oder $C_1$—$C_4$-Alkoxy bedeutet, und

$R^3$ und $R^4$ und $R^5$ unabhängig (1) Wasserstoff, (2) Halogen, (3) $C_1$—$C_4$-Alkyl, (4) $C_1$—$C_4$-Alkoxy, (5) Nitro, (6) $C_1$—$C_4$-Haloalkyl, (7) $R^bSO_n$—, worin $R^b$ für $C_1$—$C_4$-Alkyl steht und n eine ganze Zahl von 0, 1 oder 2 bedeutet, (8)

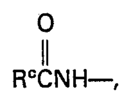

worin $R^c$ für $C_1$—$C_4$-Alkyl steht, bedeuten, (9) $R^3$ und $R^4$ zusammen mit zwei benachbarten Kohlenstoffatomen des Phenylrings eine Ringstruktur bilden,

und ihre Salze, mit der Maßgabe, daß Verbindungen der Strukturformel

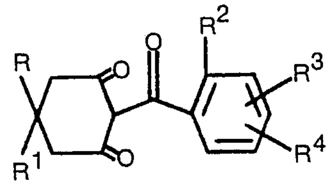

worin

R und $R^1$ Wasserstoff oder $C_1$—$C_4$-Alkyl,

$R^2$ Chlor, Brom oder Jod,

$R^3$ Wasserstoff oder Halogen, und

$R^4$ Wasserstoff, Halogen, $C_1$—$C_4$-Alkyl, $C_1$—$C_4$-Alkoxy, Nitro oder Trifluormethyl bedeuten,

ausgeschlossen sind, dadurch gekennzeichnet, daß man ein substituiertes Dion der allgemeinen Formel II

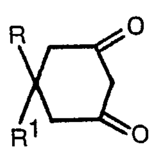

mit einem substituierten Benzolycyanid der allgemeinen Formel III

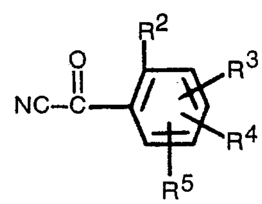

umsetzt und das Reaktionsprodukt in an sich bekannter Weise aufarbeitet.

2. Verfahren nach Anspruch 1, dadurch gekennzeichnet, daß R und $R^1$ Wasserstoff oder $C_1$—$C_4$-Alkyl; $R^2$ Chlor, Brom, Jod oder $C_1$—$C_4$-Alkoxy; $R^3$ und $R^4$ unabhängig Wasserstoff, Halogen, $C_1$—$C_4$-Alkyl, $C_1$—$C_4$-Alkoxy, Nitro, $C_1$—$C_4$-Haloalkyl, $R^bSO_n$, worin $R^b$ für $C_1$—$C_4$-Alkyl steht und n die ganze Zahl 0, 1 oder 2 bedeutet; $R^cC(O)NH$—, worin $R^c$ für $C_1$—$C_4$-Alkyl steht; $R^5$ Wasserstoff bedeuten, und ihre Salze.

3. Verfahren nach Anspruch 1, dadurch gekennzeichnet, daß R und $R^1$ Wasserstoff oder Methyl; $R^2$ Chlor, Brom oder Methoxy; $R^3$ Wasserstoff, Chlor, Dimethylamino oder Methoxy; $R^4$ Wasserstoff, Chlor, Nitro, $R^bSO_n$, worin $R^b$ für $C_1$—$C_4$-Alkyl steht und n 0 oder 2 bedeutet, $SO_2N(CH_3)_2$ oder $CF_3$; und $R^5$ Wasserstoff bedeuten, und ihre Salze.

4. Verfahren nach Anspruch 3, dadurch gekennzeichnet, daß $R^3$ in der 3-Stellung substituiert ist, $R^4$ in der 4-Stellung substituiert ist, und ihre Salze.

5. Verfahren nach Anspruch 3, dadurch gekennzeichnet, daß R und $R^1$ Wasserstoff; $R^2$ Chlor oder Methoxy; $R^3$ Wasserstoff, 3-Chlor, 3-Methoxy, 3-Ethoxy, 3-n-Propoxy, 4-Methoxy oder 3-Dimethylamino und $R^4$ 4-Chlor, 4-Brom, 4-$CH_3SO_2$—, 4$C_2H_5SO_2$— oder 4-$CF_3SO_2$ und $R^5$ Wasserstoff bedeuten, und ihre Salze.

6. Verfahren nach Anspruch 2, dadurch gekennzeichnet, daß Salze der Verbindungen, worin $R^2$ Chlor bedeutet, hergestellt werden.

7. Verfahren nach Anspruch 1, dadurch gekennzeichnet, daß Salze der Verbindungen, worin R Methyl, $R^1$ Methyl, $R^2$ Chlor, $R^3$ Wasserstoff, $R^4$ Wasserstoff und $R^5$ 6-Chlor bedeuten, hergestellt werden.

8. Verfahren nach Anspruch 1, dadurch gekennzeichnet, daß Salze der Verbindung, worin R Wasserstoff, $R^1$ Wasserstoff, $R^2$ Chlor, $R^3$ Wasserstoff, $R^4$ Wasserstoff, $R^5$ 6-Chlor bedeuten, hergestellt werden.

9. Verfahren nach Anspruch 1, dadurch gekennzeichnet, daß Salze der Verbindung, worin R Wasserstoff, $R^1$ Wasserstoff, $R^2$ Chlor, $R^3$ Wasserstoff, $R^4$ 4-Chlor und $R^5$ Wasserstoff bedeuten, hergestellt werden.

10. Verfahren nach Anspruch 1, dadurch gekennzeichnet, daß Salze der Verbindung, worin R Wasserstoff, $R^1$ Wasserstoff, $R^2$ Chlor, $R^3$ 3-Chlor und $R^4$ Wasserstoff und $R^5$ 6-Chlor bedeuten, hergestellt werden.

11. Verfahren nach Anspruch 1, dadurch gekennzeichnet, daß Salze der Verbindung, worin R Wasserstoff, $R^1$ Wasserstoff, $R^2$ Chlor, $R^3$ Wasserstoff, $R^4$ Wasserstoff und $R^5$ 5-Trifluormethyl bedeuten, hergestellt werden.

12. Verfahren nach Anspruch 1, dadurch gekennzeichnet, daß Salze der Verbindung, worin R Wasserstoff, $R^1$ Wasserstoff, $R^2$ Chlor, $R^3$ 3-Chlor, $R^4$ 4-Chlor und $R^5$ Wasserstoff bedeuten, hergestellt werden.

13. Verfahren nach Anspruch 1, dadurch gekennzeichnet, daß Salze der Verbindung, worin R Wasserstoff, $R^1$ Wasserstoff, $R^2$ Chlor, $R^3$ 3-Chlor, $R^4$ 4-Methoxy und $R^5$ Wasserstoff bedeuten, hergestellt werden.

14. Verfahren nach Anspruch 1, dadurch gekennzeichnet, daß Salze der Verbindung, worin R Wasserstoff, $R^1$ Wasserstoff, $R^2$ Chlor, $R^3$ Wasserstoff, $R^4$ 4-Brom und $R^5$ Wasserstoff bedeuten, hergestellt werden.

15. Verfahren nach Anspruch 1, dadurch gekennzeichnet, daß R Wasserstoff, $R^1$ Wasserstoff, $R^2$ Chlor, $R^3$ 3-Chlor, $R^4$ $nC_3H_7SO_2$— und $R^5$ Wasserstoff bedeuten, und ihre Salze.

16. Verfahren nach Anspruch 1, dadurch gekennzeichnet, daß Salze der Verbindung, worin R Isopropyl, $R^1$ Wasserstoff, $R^2$ Chlor, $R^3$ Wasserstoff, $R^4$ 4-Chlor und $R^5$ Wasserstoff bedeuten, hergestellt werden.

17. Verfahren nach Anspruch 1, dadurch gekennzeichnet, daß R Isopropyl, $R^1$ Wasserstoff, $R^2$ Chlor, $R^3$ 3-$CH_3$, $R^4$ 4-$CH_3SO_2$ und $R^5$ Wasserstoff bedeuten, und ihre Salze.

18. Verfahren nach Anspruch 1, dadurch gekennzeichnet, daß R Wasserstoff, $R^1$ Wasserstoff, $R^2$ Chlor, $R^3$ Wasserstoff, $R^4$ 4-$CH_3SO_2$ und $R^5$ Wasserstoff bedeuten, und ihre Salze.

19. Verfahren nach Anspruch 12, dadurch gekennzeichnet, daß die Natrium-, Isopropylamin-, Triethylamin- und Kaliumsalze hergestellt werden.

20. Verfahren zur Kontrolle unerwünschter Vegetation, dadurch gekennzeichnet, daß man auf die Fläche, wo eine Kontrolle erfolgen soll, eine herbizid wirksame Menge einer Verbindung der Formel

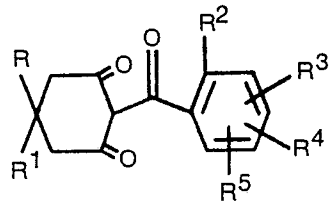

worin

R und $R^1$ Wasserstoff, $C_1$—$C_4$-Alkyl, $R^aOC(O)$—, worin $R^a$ für $C_1$—$C_4$-Alkyl steht, bedeuten,

$R^2$ Chlor, Brom, Jod oder $C_1$—$C_4$-Alkoxy bedeutet, und

$R^3$ und $R^4$ und $R^5$ unabhängig (1) Wasserstoff, (2) Halogen, (3) $C_1$—$C_4$-Alkyl, (4) $C_1$—$C_4$-Alkoxy, (5) Nitro, (6) $C_1$—$C_4$-Haloalkyl, (7) $R^bSO_n$—, worin $R^b$ für $C_1$—$C_4$-Alkyl steht und n eine ganze Zahl von 0, 1 oder 2 bedeutet, (8)

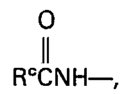

worin $R^c$ für $C_1$—$C_4$-Alkyl steht, bedeuten, (9) $R^3$ und $R^4$ zusammen mit zwei benachbarten Kohlenstoffatomen des Phenylrings eine Ringstruktur bilden
oder eines ihrer Salze anwendet, mit der Maßgabe, daß Verbindungen der Strukturformel

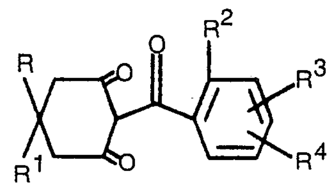

worin
R und $R^1$ Wasserstoff oder $C_1$—$C_4$-Alkyl,
$R^2$ Chlor, Brom oder Jod,
$R^3$ Wasserstoff oder Halogen, und
$R^4$ Wasserstoff, Halogen, $C_1$—$C_4$-Alkyl, $C_1$—$C_4$-Alkoxy, Nitro oder Trifluormethyl bedeuten,
ausgeschlossen sind.

21. Verfahren nach Anspruch 20, dadurch gekennzeichnet, daß R und $R^1$ Wasserstoff oder $C_1$—$C_4$-Alkyl; $R^2$ Chlor, Brom, Jod oder $C_1$—$C_4$-Alkoxy; $R^3$ und $R^4$ unabhängig Wasserstoff, Halogen, $C_1$—$C_4$-Alkyl, $C_1$—$C_4$-Alkoxy, Nitro, $C_1$—$C_4$-Haloalkyl, $R^bSO_n$, worin $R^b$ für $C_1$—$C_4$-Alkyl steht und n die ganze Zahl 0, 1 oder 2 bedeutet; $R^cC(O)NH$—, worin $R^c$ für $C_1$—$C_4$-Alkyl steht; $R^5$ Wasserstoff bedeuten, und ihre Salze.

22. Verfahren nach Anspruch 20, dadurch gekennzeichnet, daß R und $R^1$ Wasserstoff oder Methyl; $R^2$ Chlor, Brom oder Methoxy; $R^3$ Wasserstoff, Chlor oder Methoxy; $R^4$ Wasserstoff, Chlor, Nitro, $R^bSO_n$, worin $R^b$ für $C_1$—$C_4$-Alkyl steht und n 0 oder 2 bedeutet, $SO_2N(CH_3)_2$ oder $CF_3$; und $R^5$ Wasserstoff bedeuten, und ihre Salze.

23. Verfahren nach Anspruch 22, dadurch gekennzeichnet, daß $R^3$ in der 3-Stellung substituiert ist, $R^4$ in der 4-Stellung substituiert ist und $R^5$ in der 5- oder 6-Stellung substituiert ist, und ihre Salze.

24. Verfahren nach Anspruch 22, dadurch gekennzeichnet, daß R und $R^1$ Wasserstoff; $R^2$ Chlor oder Methoxy; $R^3$ Wasserstoff, 3-Chlor, 3-Methoxy, 3-Ethoxy, 3-n-Propoxy, 4-Methoxy oder 3-Dimethylamino und $R^4$ 4-Chlor, 4-Brom, 4-$CH_3SO_2$—, $4C_2H_5SO_2$— oder 4-$CF_3SO_2$ und $R^5$ Wasserstoff bedeuten, und ihre Salze.

25. Verfahren nach Anspruch 24, dadurch gekennzeichnet, daß $R^2$ Chlor bedeutet, und ihre Salze.

26. Verfahren nach Anspruch 20, dadurch gekennzeichnet, daß ein Salz einer Verbindung, worin R Methyl, $R^1$ Methyl, $R^2$ Chlor, $R^3$ Wasserstoff, $R^4$ Wasserstoff und $R^5$ 6-Chlor bedeuten, angewendet wird.

27. Verfahren nach Anspruch 20, dadurch gekennzeichnet, daß ein Salz einer Verbindung, worin R Wasserstoff, $R^1$ Wasserstoff, $R^2$ Chlor, $R^3$ Wasserstoff, $R^4$ Wasserstoff, $R^5$ 6-Chlor bedeuten, angewendet wird.

28. Verfahren nach Anspruch 20, dadurch gekennzeichnet, daß ein Salz einer Verbindung, worin R Wasserstoff, $R^1$ Wasserstoff, $R^2$ Chlor, $R^3$ Wasserstoff, $R^4$ 4-Chlor und $R^5$ Wasserstoff bedeuten, angewendet wird.

29. Verfahren nach Anspruch 20, dadurch gekennzeichnet, daß ein Salz einer Verbindung, worin R Wasserstoff, $R^1$ Wasserstoff, $R^2$ Chlor, $R^3$ 3-Chlor und $R^4$ Wasserstoff und $R^5$ 6-Chlor bedeuten, angewendet wird.

30. Verfahren nach Anspruch 20, dadurch gekennzeichnet, daß ein Salz einer Verbindung, worin R Wasserstoff, $R^1$ Wasserstoff, $R^2$ Chlor, $R^3$ Wasserstoff, $R^4$ Wasserstoff und $R^5$ 5-Trifluormethyl bedeuten, angewendet wird.

31. Verfahren nach Anspruch 20, dadurch gekennzeichnet, daß ein Salz einer Verbindung, worin R Wasserstoff, $R^1$ Wasserstoff, $R^2$ Chlor, $R^3$ 3-Chlor, $R^4$ 4-Chlor und $R^5$ Wasserstoff bedeuten, angewendet wird.

32. Verfahren nach Anspruch 20, dadurch gekennzeichnet, daß ein Salz einer Verbindung, worin R Wasserstoff, $R^1$ Wasserstoff, $R^2$ Chlor, $R^3$ 3-Chlor, $R^4$ 4-Methoxy und $R^5$ Wasserstoff bedeuten, angewendet wird.

33. Verfahren nach Anspruch 20, dadurch gekennzeichnet, daß ein Salz einer Verbindung, worin R Wasserstoff, $R^1$ Wasserstoff, $R^2$ Chlor, $R^3$ Wasserstoff, $R^4$ 4-Brom und $R^5$ Wasserstoff bedeuten, angewendet wird.

34. Verfahren nach Anspruch 20, dadurch gekennzeichnet, daß R Wasserstoff, $R^1$ Wasserstoff, $R^2$ Chlor, $R^3$ 3-Chlor, $R^4$ $nC_3H_7SO_2$— und $R^5$ Wasserstoff bedeuten, und ihre Salze.

35. Verfahren nach Anspruch 20, dadurch gekennzeichnet, daß R Isopropyl, $R^1$ Wasserstoff, $R^2$ Chlor, $R^3$ Wasserstoff, $R^4$ 4-Chlor und $R^5$ Wasserstoff bedeuten, und ihre Salze.

36. Verfahren nach Anspruch 20, dadurch gekennzeichnet, daß R Wasserstoff, $R^1$ Wasserstoff, $R^2$ Chlor, $R^3$ 3-$CH_3O$, $R^4$ 4-$CH_3SO_2$, Chlor und $R^5$ Wasserstoff bedeuten, und ihre Salze.

37. Verfahren nach Anspruch 20, dadurch gekennzeichnet, daß R Wasserstoff, $R^1$ Wasserstoff, $R^2$ Chlor, $R^3$ Wasserstoff, $R^4$ 4-$CH_3SO_2$ und $R^5$ Wasserstoff bedeuten, und ihre Salze.

38. Verfahren nach Anspruch 20, dadurch gekennzeichnet, daß die Salze die Natrium-, Isopropylamin-, Triethylamin- und Kaliumsalze sind.

39. Verfahren nach Anspruch 1, dadurch gekennzeichnet, daß R Wasserstoff, $R^1$ Wasserstoff, $R^2$ Chlor, $R^3$ 3-$C_2H_5S$, $R^4$ 4-$C_2H_5SO_2$ und $R^5$ Wasserstoff bedeuten, und ihre Salze.

40. Verfahren nach Anspruch 1, dadurch gekennzeichnet, daß R Wasserstoff, $R^1$ Wasserstoff, $R^2$ Chlor, $R^3$ 3-Methoxy, $R^4$ 4-$CH_3SO_2$ und $R^5$ Wasserstoff bedeuten, und ihre Salze.

41. Verfahren nach Anspruch 1, dadurch gekennzeichnet, daß R Wasserstoff, $R^1$ Wasserstoff, $R^2$ Chlor, $R^3$ 3-Chlor, $R^4$ 4-n-$C_3H_7SO_2$— und $R^5$ Wasserstoff bedeuten, und ihre Salze.

42. Verfahren nach Anspruch 20, dadurch gekennzeichnet, daß R Wasserstoff, $R^1$ Wasserstoff, $R^2$ Chlor, $R^3$ 3-$C_2H_5S$, $R^4$ 4-$C_2H_5SO_2$ und $R^5$ Wasserstoff bedeuten, und ihre Salze.

43. Verfahren nach Anspruch 20, dadurch gekennzeichnet, daß R Wasserstoff, $R^1$ Wasserstoff, $R^2$ Chlor, $R^3$ 3-Methoxy, $R^4$ 4-$CH_3SO_2$ und $R^5$ Wasserstoff bedeuten, und ihre Salze.

44. Verfahren nach Anspruch 20, dadurch gekennzeichnet, daß R Wasserstoff, $R^1$ Wasserstoff, $R^2$ Chlor, $R^3$ 3-Chlor, $R^4$ 4-n-$C_3H_7SO_2$— und $R^5$ Wasserstoff bedeuten, und ihre Salze.

45. Verfahren zur Herstellung einer herbiziden Zubereitung, dadurch gekennzeichnet, daß man die Verbindung nach Anspruch 18 oder eines ihrer Salze und einen inerten Träger dafür vermischt.

46. Verfahren zur Kontrolle unerwünschter Vegetation, dadurch gekennzeichnet, daß man auf die Fläche, wo eine Kontrolle erfolgen soll, eine herbizid wirksame Menge der Verbindung nach Anspruch 18 oder eines ihrer Salze und einen inerten Träger dafür anwendet.

**Revendications pour les Etats contractants: BE GB FR DE NL IT SE CH LI**

1. Un dérivé de formule structurelle:

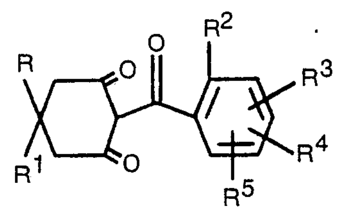

dans laquelle:

R et $R_1$ représentent un atome d'hydrogène, un radical alkyle en $C_1$ à $C_4$, un groupe $R^aOC(O)$— dans lequel $R^a$ représente un radical alkyle en $C_1$ à $C_4$;

$R^2$ représente un atome de chlore, de brome, d'iode ou un radical alkoxy en $C_1$ à $C_4$; et

$R^3$ et $R^4$ et $R^5$ représentent indépendamment: (1) un atome d'hydrogène, (2) un atome d'halogène, (3) un radical alkyle en $C_1$ à $C_4$, (4) un radical alkoxy en $C_1$ à $C_4$, (5) un groupe nitro, (6) un groupe haloalkyle en $C_1$ à $C_4$, (7) le groupe $R^bSO_n$— dans lequel le groupe $R^b$ représente un radical alkyle en $C_1$ à $C_4$ et n représente un nombre entier égal à 0, 1 ou 2, (8)

$$R^cC(=O)NH—$$

dans lequel $R^c$ représente un radical alkyle en $C_1$ à $C_4$, ou (9) $R^3$ et $R^4$ peuvent former ensemble une structure annulaire renfermant deux atomes de carbone adjacents dans le noyau du phényle,

et leurs sels sous réserve que soient exclus les dérivés de formule structurelle:

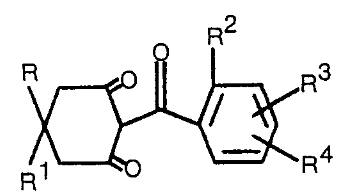

dans laquelle:

R et $R^1$ représentent un atome d'hydrogène ou un radical alkyle en $C_1$ à $C_4$,

$R^2$ représente un atome de chlore, de brome ou d'iode,

$R^3$ représente un atome d'hydrogène ou d'halogène, et

$R^4$ représente un atome d'hydrogène, d'halogène, un radical alkyle en $C_1$ à $C_4$, alkoxy en $C_1$ à $C_4$, un groupe nitro ou le radical trifluorométhyle.

2. Les dérivés selon la revendication 1, dans lesquels R et $R^1$ représentent un atome d'hydrogène ou un radical alkyle en $C_1$ à $C_4$; $R^2$ représente un atome de chlore, de brome, d'iode ou un radical alkoxy en $C_1$ à

$C_4$; $R^3$ et $R^4$ représentent indépendamment un atome d'hydrogène, d'halogène, un radical alkyle en $C_1$ à $C_4$, alkoxy en $C_1$ à $C_4$, un groupe nitro, un radical haloalkyle en $C_1$ à $C_4$, un groupe $R^bSO_n$ dans lequel $R^b$ représente un radical alkyle en $C_1$ à $C_4$ et n est un nombre entier égal à 0, 1 ou 2, $R^cC(O)NH$— dans lequel $R^c$ représente un radical alkyle en $C_1$ à $C_4$; $R^5$ représente un atome d'hydrogène; et leurs sels.

3. Les dérivés selon la revendication 1, dans lesquels R et $R^1$ représentent un atome d'hydrogène ou le radical méthyle; $R^2$ représente un atome de chlore, de brome ou le radical méthoxy; $R^3$ représente un atome d'hydrogène, de chlore, le groupe diméthylamino ou méthoxy; $R^4$ représente un atome d'hydrogène, de chlore, le groupe nitro, $R^bSO_n$ dans lequel $R^b$ représente un radical alkyle en $C_1$ à $C_4$ et n est égal à 0 ou 2, $SO_2N(CH_3)_2$ ou $CF_3$; et $R^5$ représente un atome d'hydrogène; et leurs sels.

4. Les dérivés selon la revendication 3, dans lesquels $R^3$ est substitué en position 3, $R^4$ est substitué en position 4 et $R^5$ est substitué en position 5 ou 6, et leurs sels.

5. Les dérivés selon la revendication 3, dans lesquels R et $R^1$ représentent un atome d'hydrogène; $R^2$ représente un atome de chlore ou le radical méthoxy; $R^3$ représente un atome d'hydrogène, un atome de chlore-3, le radical 3-méthoxy, 3-éthoxy, 3-n-propoxy, 4-méthoxy ou 3-diméthylamino et $R^4$ représente un atome de chlore-4, brome-4, 4-$CH_2SO_2$—, 4$C_2H_5SO_2$— ou 4-$CF_3SO_2$; et $R^5$ représente un atome d'hydrogène; et leurs sels.

6. Les sels des dérivés selon la revendication 2, dans lesquels $R^2$ représente un atome de chlore.

7. Les sels des dérivés selon la revendication 1, dans lesquels R représente le radical méthyle, $R^1$ représente le radical méthyle, $R^2$ représente un atome de chlore, $R^3$ représente un atome d'hydrogène, $R^4$ représente un atome d'hydrogène et $R^5$ représente un atome de chlore-6.

8. Les sels des dérivés selon la revendication 1, dans lesquels R représente un atome d'hydrogène, $R^1$ représente un atome d'hydrogène, $R^2$ représente un atome de chlore, $R^3$ représente un atome d'hydrogène, $R^4$ représente un atome d'hydrogène, $R^5$ représente un atome de chlore-6.

9. Les sels des dérivés selon la revendication 1, dans lesquels R représente un atome d'hydrogène, $R^1$ représente un atome d'hydrogène, $R^2$ représente un atome de chlore, $R^3$ représente un atome d'hydrogène, $R^4$ représente un atome de chlore-4 et $R^5$ représente un atome d'hydrogène.

10. Les sels des dérivés selon la revendication 1, dans lesquels R représente un atome d'hydrogène, $R^1$ représente un atome d'hydrogène, $R^2$ représente un atome de chlore, $R^3$ représente un atome de chlore-3 et $R^4$ représente un atome d'hydrogène et $R^5$ représente un atome de chlore-6.

11. Les sels des dérivés selon la revendication 1, dans lesquels R représente un atome d'hydrogène, $R^1$ représente un atome d'hydrogène, $R^2$ représente un atome de chlore, $R^3$ représente un atome d'hydrogène, $R^4$ représente un atome d'hydrogène et $R^5$ représente le radical 5-trifluorométhyle.

12. Les sels des dérivés selon la revendication 1, dans lesquels R représente un atome d'hydrogène, $R^1$ représente un atome d'hydrogène, $R^2$ représente un atome de chlore, $R^3$ représente un atome de chlore-3, $R^4$ représente un atome de chlore-4 et $R^5$ représente un atome d'hydrogène.

13. Les sels des dérivés selon la revendication 1, dans lesquels R représente un atome d'hydrogène, $R^1$ représente un atome d'hydrogène, $R^2$ représente un atome de chlore, $R^3$ représente un atome de chlore-3, $R^4$ représente le radical 4-méthoxy et $R^5$ représente un atome d'hydrogène.

14. Les sels des dérivés selon la revendication 1, dans lesquels R représente un atome d'hydrogène, $R^1$ représente un atome d'hydrogène, $R^2$ représente un atome de chlore, $R^3$ représente un atome d'hydrogène, $R^4$ représente un atome de brome-4 et $R^5$ représente un atome d'hydrogène.

15. Le dérivé selon la revendication 1, dans lesquels R représente un atome d'hydrogène, $R^1$ représente un atome d'hydrogène, $R^2$ représente un atome de chlore, $R^3$ représente un atome de chlore-3, $R^4$ représente $nC_3H_7SO_2$— et $R^5$ représente un atome d'hydrogène, et leurs sels.

16. Les sels des dérivés selon la revendication 1, dans lesquels R représente un radical isopropyle, $R^1$ représente un atome d'hydrogène, $R^2$ représente un atome de chlore, $R^3$ représente un atome d'hydrogène, $R^4$ représente un atome de chlore-4 et $R^5$ représente un atome d'hydrogène.

17. Le dérivé selon la revendication 1, dans lesquels R représente le radical isopropyle, $R^1$ représente un atome d'hydrogène, $R^2$ représente un atome de chlore, $R^3$ représente 3-$CH_3$, $R^4$ représente 4-$CH_3SO_2$ et $R^5$ représente un atome d'hydrogène, et leurs sels.

18. Le dérivé selon la revendication 1, dans lesquels R représente un atome d'hydrogène, $R^1$ représente un atome d'hydrogène, $R^2$ représente un atome de chlore, $R^3$ représente un atome d'hydrogène, $R^4$ représente 4-$CH_3SO_2$ et $R^5$ représente un atome d'hydrogène, et leurs sels.

19. Les sels selon la revendication 12, respectivement les sels de sodium, d'isopropylamine, de triéthylamine et de potassium.

20. Le procédé de contrôle d'une végétation indésirable consistant à appliquer à la zone que l'on souhaite contrôler une quantité efficace en tant qu'herbicide d'un dérivé de formule:

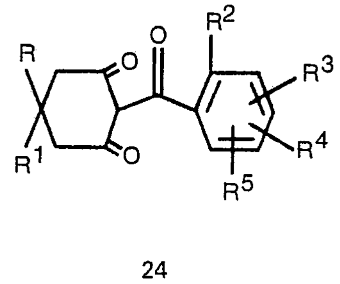

dans laquelle:

R et $R_1$ représentent un atome d'hydrogène, un radical alkyle en $C_1$ à $C_4$, un groupe $R^aOC(O)—$ dans lequel $R^a$ représente un radical alkyle en $C_1$ à $C_4$;

$R^2$ représente un atome de chlore, de brome, d'iode ou un radical alkoxy en $C_1$ à $C_4$; et

$R^3$ et $R^4$ et $R^5$ représentent indépendamment: (1) un atome d'hydrogène, (2) un atome d'halogène, (3) un radical alkyle en $C_1$ à $C_4$, (4) un radical alkoxy en $C_1$ à $C_4$, (5) un groupe nitro, (6) un groupe haloalkyle en $C_1$ à $C_4$, (7) le groupe $R^bSO_n—$ dans lequel le groupe $R^b$ représente un radical alkyle en $C_1$ à $C_4$ et n représente un nombre entier égal à 0, 1 ou 2, (8)

$$R^{c}\overset{\overset{O}{\|}}{C}NH—$$

dans lequel $R^c$ représente un radical alkyle en $C_1$ à $C_4$, ou (9) $R^3$ et $R^4$ peuvent former ensemble une structure annulaire renfermant deux atomes de carbone adjacents dans le noyau du phényle,

et leurs sels sous réserve que soient exclus les dérivés de formule structurelle:

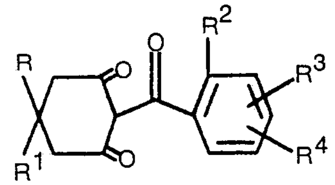

dans laquelle:

R et $R^1$ représentent un atome d'hydrogène ou un radical alkyle en $C_1$ à $C_4$,

$R^2$ représente un atome de chlore, de brome ou d'iode,

$R^3$ représente un atome d'hydrogène ou d'halogène, et

$R^4$ représente un atome d'hydrogène, d'halogène, un radical alkyle en $C_1$ à $C_4$, alkoxy en $C_1$ à $C_4$, un groupe nitro ou le radical trifluorométhyle.

21. Le procédé selon la revendication 20, dans lequel R et $R^1$ représentent un atome d'hydrogène ou un radical alkyle en $C_1$ à $C_4$; $R^2$ représente un atome de chlore, de brome, d'iode ou un radical alkoxy en $C_1$ à $C_4$; $R^3$ et $R^4$ représentent indépendamment un atome d'hydrogène, d'halogène, un radical alkyle en $C_1$ à $C_4$, alkoxy en $C_1$ à $C_4$, un groupe nitro, un radical haloalkyle en $C_1$ à $C_4$, un groupe $R^bSO_n$ dans lequel $R^b$ représente un radical alkyle en $C_1$ à $C_4$ et n est un nombre entier égal à 0, 1 ou 2, $R^cC(O)NH—$ dans lequel $R^c$ représente un radical alkyle en $C_1$ à $C_4$; $R^5$ représente un atome d'hydrogène; et leurs sels.

22. Le procédé selon la revendication 20, dans lequel R et $R^1$ représentent un atome d'hydrogène ou le radical méthyle; $R^2$ représente un atome de chlore, de brome ou le radical méthoxy; $R^3$ représente un atome d'hydrogène, de chlore, le groupe méthoxy; $R^4$ représente un atome d'hydrogène, de chlore, le groupe nitro, $R^bSO_n$ dans lequel $R^b$ représente un radical alkyle en $C_1$ à $C_4$ et n est égal à 0 ou 2, $SO_2N(CH_3)_2$ ou $CF_3$; et $R^5$ représente un atome d'hydrogène; et leurs sels.

23. Le procédé selon la revendication 22, dans lequel $R^3$ est substitué en position 3, $R^4$ est substitué en position 4 et $R^5$ est substitué en position 5 ou 6, et leurs sels.

24. Le procédé selon la revendication 22, dans lequel R et $R^1$ représentent un atome d'hydrogène; $R^2$ représente un atome de chlore ou le radical méthoxy; $R^3$ représente un atome d'hydrogène, un atome de chlore-3, le radical 3-méthoxy, 3-éthoxy, 3-n-propoxy, 4-méthoxy ou 3-diméthylamino; $R^4$ représente un atome de chlore-4, brome-4, $4-CH_2SO_2—$, $4C_2H_5SO_2—$ ou $4-CF_3SO_2$; et $R^5$ représente un atome d'hydrogène; et leurs sels.

25. Le procédé selon la revendication 24, dans lequel $R^2$ représente un atome de chlore, et leurs sels.

26. Le procédé selon la revendication 20, dans lequel on applique un sel d'un dérivé dans lequel R représente le radical méthyle, $R^1$ représente le radical méthyle, $R^2$ représente un atome de chlore, $R^3$ représente un atome d'hydrogène, $R^4$ représente un atome d'hydrogène et $R^5$ représente un atome de chlore-6.

27. Le procédé selon la revendication 20, dans lequel on applique un sel d'un dérivé dans lequel R représente un atome d'hydrogène, $R^1$ représente un atome d'hydrogène, $R^2$ représente un atome de chlore, $R^3$ représente un atome d'hydrogène, $R^4$ représente un atome d'hydrogène, $R^5$ représente un atome de chlore-6.

28. Le procédé selon la revendication 20, dans lequel on applique un sel d'un dérivé dans lequel R représente un atome d'hydrogène, $R^1$ représente un atome d'hydrogène, $R^2$ représente un atome de chlore, $R^3$ représente un atome d'hydrogène, $R^4$ représente un atome de chlore-4 et $R^5$ représente un atome d'hydrogène.

29. Le procédé selon la revendication 20, dans lequel on applique un sel d'un dérivé dans lequel R représente un atome d'hydrogène, $R^1$ représente un atome d'hydrogène, $R^2$ représente un atome de chlore, $R^3$ représente un atome de chlore-3 et $R^4$ représente un atome d'hydrogène et $R^5$ représente un atome de chlore-6.

30. Le procédé selon la revendication 20, dans lequel on applique un sel d'un dérivé dans lequel R

représente un atome d'hydrogène, $R^1$ représente un atome d'hydrogène, $R^2$ représente un atome de chlore, $R^3$ représente un atome d'hydrogène, $R^4$ représente un atome d'hydrogène et $R^5$ représente le radical 5-trifluorométhyle.

31. Le procédé selon la revendication 20, dans lequel on applique un sel d'un dérivé dans lequel R représente un atome d'hydrogène, $R^1$ représente un atome d'hydrogène, $R^2$ représente un atome de chlore, $R^3$ représente un atome de chlore-3, $R^4$ représente un atome de chlore-4 et $R^5$ représente un atome d'hydrogène.

32. Le procédé selon la revendication 20, dans lequel on applique un sel d'un dérivé dans lequel R représente un atome d'hydrogène, $R^1$ représente un atome d'hydrogène, $R^2$ représente un atome de chlore, $R^3$ représente un atome de chlore-3, $R^4$ représente le radical 4-méthoxy et $R^5$ représente un atome d'hydrogène.

33. Le procédé selon la revendication 20, dans lequel on applique un sel d'un dérivé dans lequel R représente un atome d'hydrogène, $R^1$ représente un atome d'hydrogène, $R^2$ représente un atome de chlore, $R^3$ représente un atome d'hydrogène, $R^4$ représente un atome de brome-4 et $R^5$ représente un atome d'hydrogène.

34. Le procédé selon la revendication 20, dans lequel R représente un atome d'hydrogène, $R^1$ représente un atome d'hydrogène, $R^2$ représente un atome de chlore, $R^3$ représente un atome de chlore-3, $R^4$ représente $nC_3H_7SO_2$— et $R^5$ représente un atome d'hydrogène, et leurs sels.

35. Le procédé selon la revendication 20, dans lequel R représente un radical isopropyle, $R^1$ représente un atome d'hydrogène, $R^2$ représente un atome de chlore, $R^3$ représente un atome d'hydrogène, $R^4$ représente un atome de chlore-4 et $R^5$ représente un atome d'hydrogène, et leurs sels.

36. Le procédé selon la revendication 20, dans lequel R représente un atome d'hydrogène, $R^1$ représente un atome d'hydrogène, $R^2$ représente un atome de chlore, $R^3$ représente 3-$CH_3O$, $R^4$ représente 4-$CH_3SO_2$, un atome de chlore, et $R^5$ représente un atome d'hydrogène, et leurs sels.

37. Le procédé selon la revendication 20, dans lequel R représente un atome d'hydrogène, $R^1$ représente un atome d'hydrogène, $R^2$ représente un atome de chlore, $R^3$ représente un atome d'hydrogène, $R^4$ représente 4-$CH_3SO_2$ et $R^5$ représente un atome d'hydrogène, et leurs sels.

38. Le procédé selon la revendication 20, dans lequel les sels sont les sels de sodium, d'isopropylamine, de triéthylamine et de potassium.

39. Le dérivé selon la revendication 1, dans lequel R représente un atome d'hydrogène, $R^1$ représente un atome d'hydrogène, $R^2$ représente un atome de chlore, $R^3$ représente 3-$C_2H_5S$, $R^4$ représente 4-$C_2H_5SO_2$ et $R^5$ représente un atome d'hydrogène, et leurs sels.

40. Le dérivé selon la revendication 1, dans lequel R représente un atome d'hydrogène, $R^1$ représente un atome d'hydrogène, $R^2$ représente un atome de chlore, $R^3$ représente le radical 3-méthoxy, $R^4$ représente 4-$CH_3SO_2$ et $R^5$ représente un atome d'hydrogène, et leurs sels.

41. Le dérivé selon la revendication 1, dans lequel R représente un atome d'hydrogène, $R^1$ représente un atome d'hydrogène, $R^2$ représente un atome de chlore, $R^3$ représente un atome de chlore-3, $R^4$ représente 4-n-$C_3H_7SO_2$— et $R^5$ représente un atome d'hydrogène, et leurs sels.

42. Le procédé selon la revendication 20, dans lequel R représente un atome d'hydrogène, $R^1$ représente un atome d'hydrogène, $R^2$ représente un atome de chlore, $R^3$ représente 3-$C_2H_5S$, $R^4$ représente 4-$C_2H_5SO_2$ et $R^5$ représente un atome d'hydrogène, et leurs sels.

43. Le procédé selon la revendication 20, dans lequel R représente un atome d'hydrogène, $R^1$ représente un atome d'hydrogène, $R^2$ représente un atome de chlore, $R^3$ représente le radical 3-méthoxy, $R^4$ représente 4-$CH_3SO_2$ et $R^5$ représente un atome d'hydrogène, et leurs sels.

44. Le procédé selon la revendication 20, dans lequel R représente un atome d'hydrogène, $R^1$ représente un atome d'hydrogène, $R^2$ représente un atome de chlore, $R^3$ représente un atome de chlore-3, $R^4$ représente 4-n-$C_3H_7SO_2$— et $R^5$ représente un atome d'hydrogène, et leurs sels.

45. Une composition herbicide comprenant le dérivé de la revendication 18 ou l'un de ses sels et un support inerte de ceux-ci.

46. Le procédé de contrôle d'une végétation indésirable, consistant à appliquer à la zone que l'on désire contrôler une quantité efficace en tant qu'herbicide du dérivé selon la revendication 8 ou un de ses sels et un support inerte vis-à-vis de cet herbicide.

**Revendications pour l'Etat contractant: AT**

1. Un procédé de préparation de dérivés de formule structurelle:

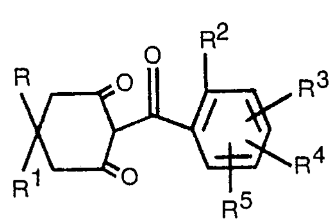

dans laquelle:

R et $R_1$ représentent un atome d'hydrogène, un radical alkyle en $C_1$ à $C_4$, un groupe $R^aOC(O)$— dans lequel $R^a$ représente un radical alkyle en $C_1$ à $C_4$;

$R^2$ représente un atome de chlore, de brome, d'iode ou un radical alkoxy en $C_1$ à $C_4$; et

$R^3$ et $R^4$ et $R^5$ représentent indépendamment: (1) un atome d'hydrogène, (2) un atome d'halogène, (3) un radical alkyle en $C_1$ à $C_4$, (4) un radical alkoxy en $C_1$ à $C_4$, (5) un groupe nitro, (6) un groupe haloalkyle en $C_1$ à $C_4$, (7) le groupe $R^bSO_n$— dans lequel le groupe $R^b$ représente un radical alkyle en $C_1$ à $C_4$ et n représente un nombre entier égal à 0, 1 ou 2, (8)

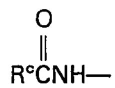

dans lequel $R^c$ représente un radical alkyle en $C_1$ à $C_4$, ou (9) $R^3$ et $R^4$ peuvent former ensemble une structure annulaire renfermant deux atomes de carbone adjacents dans le noyau du phényle, et leurs sels sous réserve que soient exclus les dérivés de formule structurelle:

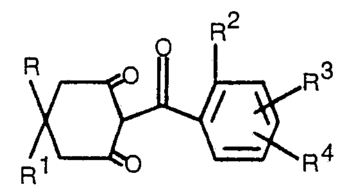

dans laquelle:

R et $R^1$ représentent un atome d'hydrogène ou un radical alkyle en $C_1$ à $C_4$,

$R^2$ représente un atome de chlore, de brome ou d'iode,

$R^3$ représente un atome d'hydrogène ou d'halogène, et

$R^4$ représente un atome d'hydrogène, d'halogène, un radical alkyle en $C_1$ à $C_4$, alkoxy en $C_1$ à $C_4$, un groupe nitro ou le radical trifluorométhyle,

consistant à faire réagir une dione substituée de formule générale II:

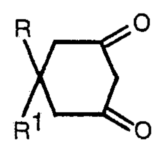

avec un cyanure de benzoyle substitué de formule générale III:

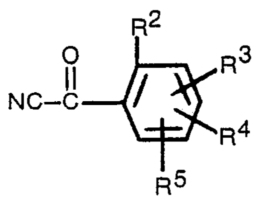

et à élaborer le produit de la réaction par des techniques classiques.

2. Le procédé selon la revendication 1, dans lequel R et $R^1$ représentent un atome d'hydrogène ou un radical alkyle en $C_1$ à $C_4$; $R^2$ représente un atome de chlore, de brome, d'iode ou un radical alkoxy en $C_1$ à $C_4$; $R^3$ et $R^4$ représentent indépendamment un atome d'hydrogène, d'halogène, un radical alkyle en $C_1$ à $C_4$, alkoxy en $C_1$ à $C_4$, un groupe nitro, un radical haloalkyle en $C_1$ à $C_4$, un groupe $R^bSO_n$ dans lequel $R^b$ représente un radical alkyle en $C_1$ à $C_4$ et n est un nombre entier égal à 0, 1 ou 2, $R^cC(O)NH$— dans lequel $R^c$ représente un radical alkyle en $C_1$ à $C_4$; $R^5$ représente un atome d'hydrogène; et leurs sels.

3. Le procédé selon la revendication 1, dans lequel R et $R^1$ représentent un atome d'hydrogène ou le radical méthyle; $R^2$ représente un atome de chlore, de brome ou le radical méthoxy; $R^3$ représente un atome d'hydrogène, de chlore, le groupe diméthylamino ou méthoxy; $R^4$ représente un atome d'hydrogène, de chlore, le groupe nitro, $R^bSO_n$ dans lequel $R^b$ représente un radical alkyle en $C_1$ à $C_4$ et n est égal à 0 ou 2, $SO_2N(CH_3)_2$ ou $CF_3$; et $R^5$ représente un atome d'hydrogène; et leurs sels.

4. Le procédé selon la revendication 3, dans lequel $R^3$ est substitué en position 3, $R^4$ est substitué en position 4 et $R^5$ est substitué en position 5 ou 6, et leurs sels.

5. Le procédé selon la revendication 3, dans lequel R et $R^1$ représentent un atome d'hydrogène; $R^2$ représente un atome de chlore ou le radical méthoxy; $R^3$ représente un atome d'hydrogène, un atome de chlore-3, le radical 3-méthoxy, 3-éthoxy, 3-n-propoxy, 4-méthoxy ou 3-diméthylamino et $R^4$ représente un atome de chlore-4, brome-4, 4-$CH_2SO_2$—, 4$C_2H_5SO_2$— ou 4-$CF_3SO_2$; et $R^5$ représente un atome d'hydrogène; et leurs sels.

6. Le procédé selon la revendication 2 dans lequel on prépare des sels des dérivés dans lequel $R^2$ représente un atome de chlore.

7. Le procédé selon la revendication 1 dans lequel on prépare des sels des dérivés dans lequel R représente le radical méthyle, $R^1$ représente le radical méthyle, $R^2$ représente un atome de chlore, $R^3$ représente un atome d'hydrogène, $R^4$ représente un atome d'hydrogène et $R^5$ représente un atome de chlore-6.

8. Le procédé selon la revendication 1, dans lequel on prépare des sels des dérivés dans lequel R représente un atome d'hydrogène, $R^1$ représente un atome d'hydrogène, $R^2$ représente un atome de chlore, $R^3$ représente un atome d'hydrogène, $R^4$ représente un atome d'hydrogène, $R^5$ représente un atome de chlore-6.

9. Le procédé selon la revendication 1, dans lequel on prépare des sels des dérivés dans lequel R représente un atome d'hydrogène, $R^1$ représente un atome d'hydrogène, $R^2$ représente un atome de chlore, $R^3$ représente un atome d'hydrogène, $R^4$ représente un atome de chlore-4 et $R^5$ représente un atome d'hydrogène.

10. Le procédé selon la revendication 1, dans lequel on prépare des sels des dérivés dans lequel R représente un atome d'hydrogène, $R^1$ représente un atome d'hydrogène, $R^2$ représente un atome de chlore, $R^3$ représente un atome de chlore-3 et $R^4$ représente un atome d'hydrogène et $R^5$ représente un atome de chlore-6.

11. Le procédé selon la revendication 1, dans lequel on prépare des sels des dérivés dans lequel R représente un atome d'hydrogène, $R^1$ représente un atome d'hydrogène, $R^2$ représente un atome de chlore, $R^3$ représente un atome d'hydrogène, $R^4$ représente un atome d'hydrogène et $R^5$ représente le radical 5-trifluorométhyle.

12. Le procédé selon la revendication 1, dans lequel on prépare des sels des dérivés dans lequel R représente un atome d'hydrogène, $R^1$ représente un atome d'hydrogène, $R^2$ représente un atome de chlore, $R^3$ représente un atome de chlore-3, $R^4$ représente un atome de chlore-4 et $R^5$ représente un atome d'hydrogène.

13. Le procédé selon la revendication 1, dans lequel on prépare des sels des dérivés dans lequel R représente un atome d'hydrogène, $R^1$ représente un atome d'hydrogène, $R^2$ représente un atome de chlore, $R^3$ représente un atome de chlore-3, $R^4$ représente le radical 4-méthoxy et $R^5$ représente un atome d'hydrogène.

14. Le procédé selon la revendication 1, dans lequel on prépare des sels des dérivés dans lequel R représente un atome d'hydrogène, $R^1$ représente un atome d'hydrogène, $R^2$ représente un atome de chlore, $R^3$ représente un atome d'hydrogène, $R^4$ représente un atome de brome-4 et $R^5$ représente un atome d'hydrogène.

15. Le procédé selon la revendication 1, dans lequel R représente un atome d'hydrogène, $R^1$ représente un atome d'hydrogène, $R^2$ représente un atome de chlore, $R^3$ représente un atome de chlore-3, $R^4$ représente $nC_3H_7SO_2$— et $R^5$ représente un atome d'hydrogène, et leurs sels.

16. Le procédé selon la revendication 1, dans lequel on prépare des sels des dérivés dans lequel R représente un radical isopropyle, $R^1$ représente un atome d'hydrogène, $R^2$ représente un atome de chlore, $R^3$ représente un atome d'hydrogène, $R^4$ représente un atome de chlore-4 et $R^5$ représente un atome d'hydrogène.

17. Le procédé selon la revendication 1, dans lequel R représente le radical isopropyle, $R^1$ représente un atome d'hydrogène, $R^2$ représente un atome de chlore, $R^3$ représente 3-$CH_3$, $R^4$ représente 4-$CH_3SO_2$ et $R^5$ représente un atome d'hydrogène, et leurs sels.

18. Le procédé selon la revendication 1, dans lequel R représente un atome d'hydrogène, $R^1$ représente un atome d'hydrogène, $R^2$ représente un atome de chlore, $R^3$ représente un atome d'hydrogène, $R^4$ représente 4-$CH_3SO_2$ et $R^5$ représente un atome d'hydrogène, et leurs sels.

19. Le procédé selon la revendication 12, dans lequel on prépare des sels de sodium, d'isopropylamine, de triéthylamine et de potassium.

20. Le procédé de contrôle d'une végétation indésirable consistant à appliquer à la zone que l'on souhaite contrôler une quantité efficace en tant qu'herbicide d'un dérivé de formule:

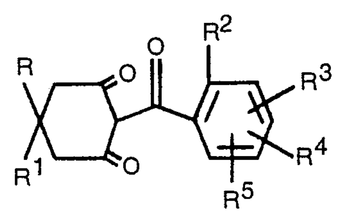

dans laquelle:

R et $R_1$ représentent un atome d'hydrogène, un radical alkyle en $C_1$ à $C_4$, un groupe $R^aOC(O)$— dans lequel $R^a$ représente un radical alkyle en $C_1$ à $C_4$;

$R^2$ représente un atome de chlore, de brome, d'iode ou un radical alkoxy en $C_1$ à $C_4$; et

$R^3$ et $R^4$ et $R^5$ représentent indépendamment: (1) un atome d'hydrogène, (2) un atome d'halogène, (3) un radical alkyle en $C_1$ à $C_4$, (4) un radical alkoxy en $C_1$ à $C_4$, (5) un groupe nitro, (6) un groupe haloalkyle en

$C_1$ à $C_4$, (7) le groupe $R^bSO_n$— dans lequel le groupe $R^b$ représente un radical alkyle en $C_1$ à $C_4$ et n représente un nombre entier égal à 0, 1 ou 2, (8)

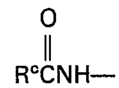

dans lequel $R^c$ représente un radical alkyle en $C_1$ à $C_4$, ou (9) $R^3$ et $R^4$ peuvent former ensemble une structure annulaire renfermant deux atomes de carbone adjacents dans le noyau du phényle, et leurs sels sous réserve que soient exclus les dérivés de formule structurelle:

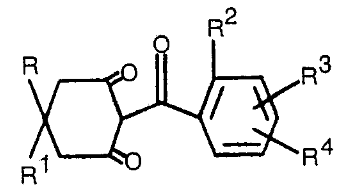

dans laquelle:

R et $R^1$ représentent un atome d'hydrogène ou un radical alkyle en $C_1$ à $C_4$,

$R^2$ représente un atome de chlore, de brome ou d'iode,

$R^3$ représente un atome d'hydrogène ou d'halogène, et

$R^4$ représente un atome d'hydrogène, d'halogène, un radical alkyle en $C_1$ à $C_4$, alkoxy en $C_1$ à $C_4$, un groupe nitro ou le radical trifluorométhyle.

21. Le procédé selon la revendication 20, dans lequel R et $R^1$ représentent un atome d'hydrogène ou un radical alkyle en $C_1$ à $C_4$; $R^2$ représente un atome de chlore, de brome, d'iode ou un radical alkoxy en $C_1$ à $C_4$; $R^3$ et $R^4$ représentent indépendamment un atome d'hydrogène, d'halogène, un radical alkyle en $C_1$ à $C_4$, alkoxy en $C_1$ à $C_4$, un groupe nitro, un radical haloalkyle en $C_1$ à $C_4$, un groupe $R^bSO_n$ dans lequel $R^b$ représente un radical alkyle en $C_1$ à $C_4$ et n est un nombre entier égal à 0, 1 ou 2, $R^cC(O)NH$— dans lequel $R^c$ représente un radical alkyle en $C_1$ à $C_4$; $R^5$ représente un atome d'hydrogène; et leurs sels.

22. Le procédé selon la revendication 20, dans lequel R et $R^1$ représentent un atome d'hydrogène ou le radical méthyle; $R^2$ représente un atome de chlore, de brome ou le radical méthoxy; $R^3$ représente un atome d'hydrogène, de chlore, le groupe méthoxy; $R^4$ représente un atome d'hydrogène, de chlore, le groupe nitro, $R^bSO_n$ dans lequel $R^b$ représente un radical alkyle en $C_1$ à $C_4$ et n est égal à 0 ou 2, $SO_2N(CH_3)_2$ ou $CF_3$; et $R^5$ représente un atome d'hydrogène; et leurs sels.

23. Le procédé selon la revendication 22, dans lequel $R^3$ est substitué en position 3, $R^4$ est substitué en position 4 et $R^5$ est substitué en position 5 ou 6, et leurs sels.

24. Le procédé selon la revendication 22, dans lequel R et $R^1$ représentent un atome d'hydrogène; $R^2$ représente un atome de chlore ou le radical méthoxy; $R^3$ représente un atome d'hydrogène, un atome de chlore-3, le radical 3-méthoxy, 3-éthoxy, 3-n-propoxy, 4-méthoxy ou 3-diméthylamino; $R^4$ représente un atome de chlore-4, brome-4, 4-$CH_2SO_2$—, 4$C_2H_5SO_2$— ou 4-$CF_3SO_2$; et $R^5$ représente un atome d'hydrogène; et leurs sels.

25. Le procédé selon la revendication 24, dans lequel $R^2$ représente un atome de chlore, et leurs sels.

26. Le procédé selon la revendication 20, dans lequel on applique un sel d'un dérivé dans lequel R représente le radical méthyle, $R^1$ représente le radical méthyle, $R^2$ représente un atome de chlore, $R^3$ représente un atome d'hydrogène, $R^4$ représente un atome d'hydrogène et $R^5$ représente un atome de chlore-6.

27. Le procédé selon la revendication 20, dans lequel on applique un sel d'un dérivé dans lequel R représente un atome d'hydrogène, $R^1$ représente un atome d'hydrogène, $R^2$ représente un atome de chlore, $R^3$ représente un atome d'hydrogène, $R^4$ représente un atome d'hydrogène, $R^5$ représente un atome de chlore-6.

28. Le procédé selon la revendication 20, dans lequel on applique un sel d'un dérivé dans lequel R représente un atome d'hydrogène, $R^1$ représente un atome d'hydrogène, $R^2$ représente un atome de chlore, $R^3$ représente un atome d'hydrogène, $R^4$ représente un atome de chlore-4 et $R^5$ représente un atome d'hydrogène.

29. Le procédé selon la revendication 20, dans lequel on applique un sel d'un dérivé dans lequel R représente un atome d'hydrogène, $R^1$ représente un atome d'hydrogène, $R^2$ représente un atome de chlore, $R^3$ représente un atome de chlore-3 et $R^4$ représente un atome d'hydrogène et $R^5$ représente un atome de chlore-6.

30. Le procédé selon la revendication 20, dans lequel on applique un sel d'un dérivé dans lequel R représente un atome d'hydrogène, $R^1$ représente un atome d'hydrogène, $R^2$ représente un atome de chlore, $R^3$ représente un atome d'hydrogène, $R^4$ représente un atome d'hydrogène et $R^5$ représente le radical 5-trifluorométhyle.

31. Le procédé selon la revendication 20, dans lequel on applique un sel d'un dérivé dans lequel R représente un atome d'hydrogène, $R^1$ représente un atome d'hydrogène, $R^2$ représente un atome de chlore,

$R^3$ représente un atome de chlore-3, $R^4$ représente un atome de chlore-4 et $R^5$ représente un atome d'hydrogène.

32. Le procédé selon la revendication 20, dans lequel on applique un sel d'un dérivé dans lequel R représente un atome d'hydrogène, $R^1$ représente un atome d'hydrogène, $R^2$ représente un atome de chlore, $R^3$ représente un atome de chlore-3, $R^4$ représente le radical 4-méthoxy et $R^5$ représente un atome d'hydrogène.

33. Le procédé selon la revendication 20, dans lequel on applique un sel d'un dérivé dans lequel R représente un atome d'hydrogène, $R^1$ représente un atome d'hydrogène, $R^2$ représente un atome de chlore, $R^3$ représente un atome d'hydrogène, $R^4$ représente un atome de brome-4 et $R^5$ représente un atome d'hydrogène.

34. Le procédé selon la revendication 20, dans lequel R représente un atome d'hydrogène, $R^1$ représente un atome d'hydrogène, $R^2$ représente un atome de chlore, $R^3$ représente un atome de chlore-3, $R^4$ représente $nC_3H_7SO_2$— et $R^5$ représente un atome d'hydrogène, et leurs sels.

35. Le procédé selon la revendication 20, dans lequel R représente un radical isopropyle, $R^1$ représente un atome d'hydrogène, $R^2$ représente un atome de chlore, $R^3$ représente un atome d'hydrogène, $R^4$ représente un atome de chlore-4 et $R^5$ représente un atome d'hydrogène, et leurs sels.

36. Le procédé selon la revendication 20, dans lequel R représente un atome d'hydrogène, $R^1$ représente un atome d'hydrogène, $R^2$ représente un atome de chlore, $R^3$ représente $3\text{-}CH_3O$, $R^4$ représente $4\text{-}CH_3SO_2$, un atome de chlore, et $R^5$ représente un atome d'hydrogène, et leurs sels.

37. Le procédé selon la revendication 20, dans lequel R représente un atome d'hydrogène, $R^1$ représente un atome d'hydrogène, $R^2$ représente un atome de chlore, $R^3$ représente un atome d'hydrogène, $R^4$ représente $4\text{-}CH_3SO_2$ et $R^5$ représente un atome d'hydrogène, et leurs sels.

38. Le procédé selon la revendication 20, dans lequel les sels sont les sels de sodium, d'isopropylamine, de triéthylamine et de potassium.

39. Le dérivé selon la revendication 1, dans lequel R représente un atome d'hydrogène, $R^1$ représente un atome d'hydrogène, $R^2$ représente un atome de chlore, $R^3$ représente $3\text{-}C_2H_5S$, $R^4$ représente $4\text{-}C_2H_5SO_2$ et $R^5$ représente un atome d'hydrogène, et leurs sels.

40. Le dérivé selon la revendication 1, dans lequel R représente un atome d'hydrogène, $R^1$ représente un atome d'hydrogène, $R^2$ représente un atome de chlore, $R^3$ représente le radical 3-méthoxy, $R^4$ représente $4\text{-}CH_3SO_2$ et $R^5$ représente un atome d'hydrogène, et leurs sels.

41. Le dérivé selon la revendication 1, dans lequel R représente un atome d'hydrogène, $R^1$ représente un atome d'hydrogène, $R^2$ représente un atome de chlore, $R^3$ représente un atome de chlore-3, $R^4$ représente $4\text{-n-}C_3H_7SO_2$— et $R^5$ représente un atome d'hydrogène, et leurs sels.

42. Le procédé selon la revendication 20, dans lequel R représente un atome d'hydrogène, $R^1$ représente un atome d'hydrogène, $R^2$ représente un atome de chlore, $R^3$ représente $3\text{-}C_2H_5S$, $R^4$ représente $4\text{-}C_2H_5SO_2$ et $R^5$ représente un atome d'hydrogène, et leurs sels.

43. Le procédé selon la revendication 20, dans lequel R représente un atome d'hydrogène, $R^1$ représente un atome d'hydrogène, $R^2$ représente un atome de chlore, $R^3$ représente le radical 3-méthoxy, $R^4$ représente $4\text{-}CH_3SO_2$ et $R^5$ représente un atome d'hydrogène, et leurs sels.

44. Le procédé selon la revendication 20, dans lequel R représente un atome d'hydrogène, $R^1$ représente un atome d'hydrogène, $R^2$ représente un atome de chlore, $R^3$ représente un atome de chlore-3, $R^4$ représente $4\text{-n-}C_3H_7SO_2$— et $R^5$ représente un atome d'hydrogène, et leurs sels.

45. Un procédé de préparation d'une composition herbicide consistant à mélanger le dérivé selon la revendication 18 ou l'un de ses sels avec un support inerte de ceux-ci.

46. Le procédé de contrôle d'une végétation indésirable, consistant à appliquer à la zone que l'on désire contrôler une quantité efficace en tant qu'herbicide du dérivé selon la revendication 18 ou l'un de ses sels et un support inerte de ceux-ci.